(19) Europäisches Patentamt
European Patent Office
Office européen des brevets

(11) Numéro de publication: **0 519 819 B1**

# (12) FASCICULE DE BREVET EUROPEEN

(45) Date de publication de fascicule du brevet: **19.07.95**

(51) Int. Cl.[6]: **A61K 7/06**, C07D 239/50

(21) Numéro de dépôt: **92401689.2**

(22) Date de dépôt: **17.06.92**

## (54) Composition pour freiner la chute des cheveux et pour induire et stimuler leur croissance à base de dérivés de pyrimidines N-oxyde trisubstitués en position 2,4 et 6 par une fonction amine, et nouveaux composés pyrimidines N-oxyde.

(30) Priorité: **20.06.91 FR 9107591**

(43) Date de publication de la demande:
**23.12.92 Bulletin 92/52**

(45) Mention de la délivrance du brevet:
**19.07.95 Bulletin 95/29**

(84) Etats contractants désignés:
**AT BE CH DE DK ES FR GB GR IT LI NL PT SE**

(56) Documents cités:
**EP-A- 0 420 707**
**WO-A-86/00616**
**WO-A-86/04231**
**US-A- 4 287 338**
**US-A- 4 308 271**

(73) Titulaire: **L'OREAL**
**14, rue Royale**
**F-75008 Paris (FR)**

(72) Inventeur: **Hocquaux, Michel**
**70, rue du Rendez-vous**
**F-75012 Paris (FR)**
Inventeur: **Bakkar, Khalid**
**20 Place Jacques Prévert**
**F-93270 Sevran (FR)**
Inventeur: **Galey, Jean-Baptiste**
**20, rue Lacépède**
**F-75005 Paris (FR)**
Inventeur: **Terranova, Eric**
**60, rue Maurice Bokanowski**
**F-92600 Asnières (FR)**

(74) Mandataire: **Casalonga, Axel et al**
**BUREAU D.A. CASALONGA - JOSSE**
**Morassistrasse 8**
**D-80469 München (DE)**

EP 0 519 819 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

L'invention concerne des compositions destinées à être utilisées, notamment en application topique, pour freiner la chute des cheveux et pour induire et stimuler leur croissance, contenant des composés pyrimidines N-oxyde trisubstitués en positions 2, 4 et 6 par une fonction amine ou leurs dérivés sulfoconjugués, ainsi que les nouveaux composés pyrimidines N-oxyde ou leurs dérivés sulfoconjugués utilisés dans ces compositions.

On connaît déjà, dans l'état de la technique, le diamino-2,4 pipéridino-6 pyrimidine oxyde-3 ou "Minoxidil" pour ses propriétés d'agent antihypertenseur, mais également pour son utilisation dans le traitement de la chute des cheveux, de la pelade, de la dermatite desquamante et de l'alopécie.

La demanderesse a découvert de nouvelles compositions pour le traitement et la prévention de la chute des cheveux, utilisées notamment en application topique, particulièrement efficaces dans le traitement des maladies du cuir chevelu grâce à une famille particulière de composés dérivés de la pyrimidine oxyde-3 trisubstitués en positions 2, 4 et 6 par une fonction amine.

Les composés retenus par la demanderesse sont particulièrement efficaces pour la repousse des cheveux et pour induire et stimuler leur croissance et freiner leur chute. Ils présentent en outre une excellente solubilité dans les milieux habituellement utilisés en cosmétique et en pharmacie.

De plus, la demanderesse a constaté que certains de ces dérivés présentent une activité antihypertensive nettement inférieure à celle du Minoxidil, voire nulle.

L'invention a donc pour objet de nouvelles compositions pour le traitement et la prévention de la chute des cheveux contenant des composés pyrimidines N-oxyde ou leurs dérivés sulfoconjugués particuliers.

L'invention a également pour objet de nouveaux composés pyrimidines N-oxyde ou leurs dérivés sulfoconjugués utilisés dans ces compositions.

Un autre objet concerne l'utilisation des composés de l'invention pour la préparation d'un médicament pour le traitement de la chute des cheveux.

D'autres objets apparaîtront à la lumière de la description et des exemples qui suivent.

Les compositions conformes à l'invention sont essentiellement caractérisées par le fait qu'elles contiennent dans un milieu physiologiquement acceptable, au moins un composé de formule suivante :

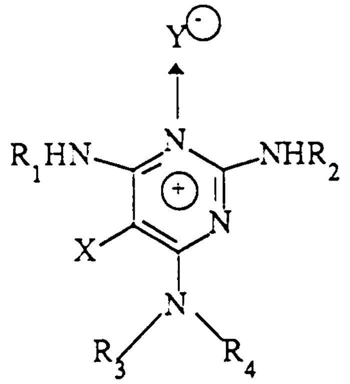

(I)

dans laquelle :

$R_1$ et $R_2$, indépendamment l'un de l'autre, désignent un atome d'hydrogène ou un radical alkyle en $C_1$-$C_8$, à condition qu'ils ne désignent pas simultanément un atome d'hydrogène;

$R_3$ et $R_4$, indépendamment l'un de l'autre, désignent un atome d'hydrogène, un radical alkyle en $C_1$-$C_8$ ou forment avec l'atome d'azote attaché à la position 6 du cycle pyrimidine, un hétérocycle en $C_3$-$C_8$, à condition qu'ils ne désignent pas simultanément un atome d'hydrogène;

X désigne un atome d'hydrogène ou un halogène;

Y désigne O ou $OSO_3$.

Les composés de formule (I), conformes à l'invention, peuvent être transformés en leurs sels d'addition d'acides cosmétiquement ou pharmaceutiquement acceptables, tels que les sels des acides sulfurique, chlorhydrique, bromhydrique, phosphorique, acétique, benzoïque, salicylique, glycolique, succinique, nicotinique, tartrique, maléique, pamoïque, méthane sulfonique, picrique, lactique, etc...

Conformément à l'invention, les radicaux alkyle en $C_1$-$C_8$ sont de préférence choisis parmi les radicaux méthyle, éthyle, n-propyle, isopropyle, n-butyle, tertiobutyle, octyle, éthyl-2 hexyle, hexyle.

Par atome d'halogène, on entend de préférence le chlore ou le brome.

Par un groupement hétérocyclique en $C_3$-$C_8$, on entend de préférence les groupements morpholino, pipéridino, pyrrolidino, pipérazino, N-alkyl-4′ pipérazino, dans lequel le groupement akyle en position 4′ contient de préférence 1 à 6 atomes de carbone.

Parmi les composés de formule générale (I), un certain nombre de composés sont connus en eux-mêmes et ont été décrits comme agents antihypertenseurs, notamment dans les brevets US-A-4.287.338 et US-A-4.308.271 (UPJOHN).

Les composés nouveaux conformes à la présente invention ont pour structure :

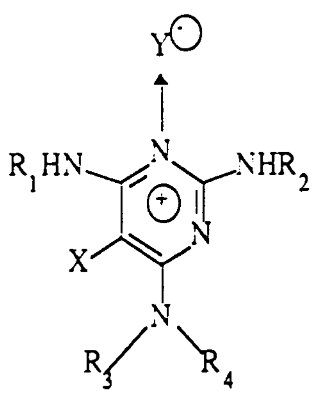

(I′)

dans laquelle $R_1$, $R_2$, $R_3$, $R_4$ et X ont les mêmes significations indiquées dans la formule (I) ci-dessus, sous réserve que $R_1$ et $R_2$ désignent simultanément un radical alkyle en $C_1$-$C_8$; et leurs sels d'addition d'acides physiologiquement acceptables.

Les composés particuliers de formule (IA) suivante :

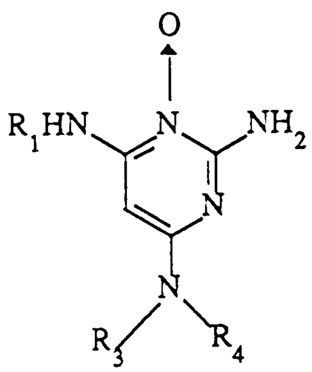

(IA)

dans laquelle $R_1$, $R_3$ et $R_4$ ont les mêmes significations indiquées ci-dessus, peuvent être obtenus en faisant réagir une amine de formule $R_1NH_2$ sur la 2-amino 4,6-dichloropyrimidine de formule :

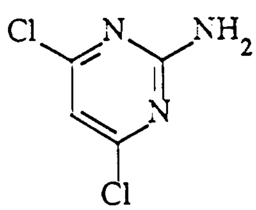

(1)

On effectue ensuite une oxydation en position 3 du noyau pyrimidine par un peracide carboxylique tel que l'acide metachloroperbenzoïque, suivie de l'introduction de l'amine $NHR_3R_4$ par substitution nucléophile aromatique sur le noyau pyrimidine du produit obtenu.

Ce procédé peut être représenté par le schéma suivant :

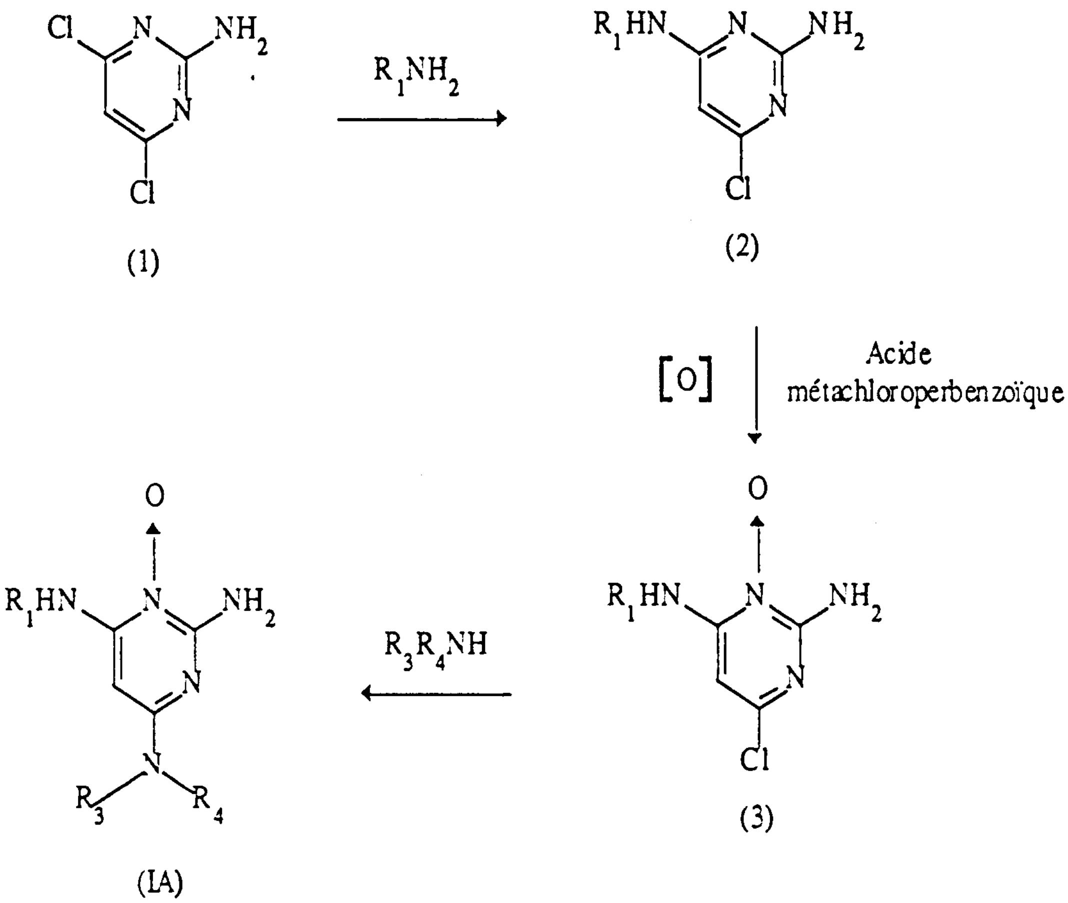


(1) (2) (3) (IA)

SCHEMA A

Les composés particuliers de formule (IB) suivante :

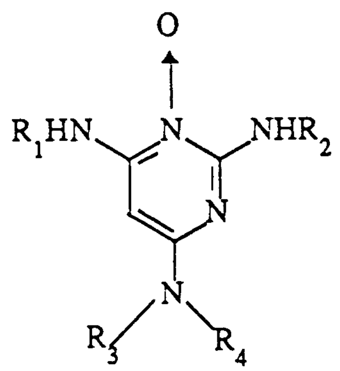

(IB)

dans laquelle $R_1$, $R_2$, $R_3$, $R_4$ ont les mêmes indications définies ci-dessus, sont obtenus en faisant réagir successivement une amine $R_1NH_2$ et une amine $R_2NH_2$ sur la trichloro-2,4,6 pyrimidine de formule (4) :

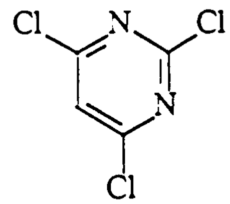

(4)

On effectue ensuite une oxydation en position 3 du noyau pyrimidine, par un peracide carboxylique, de préférence l'acide metachloroperbenzoïque, suivie d'une substitution nucléophile aromatique de l'atome de chlore en position 6 par une amine $HNR_3R_4$.

Ce procédé peut être représenté par le schéma suivant :

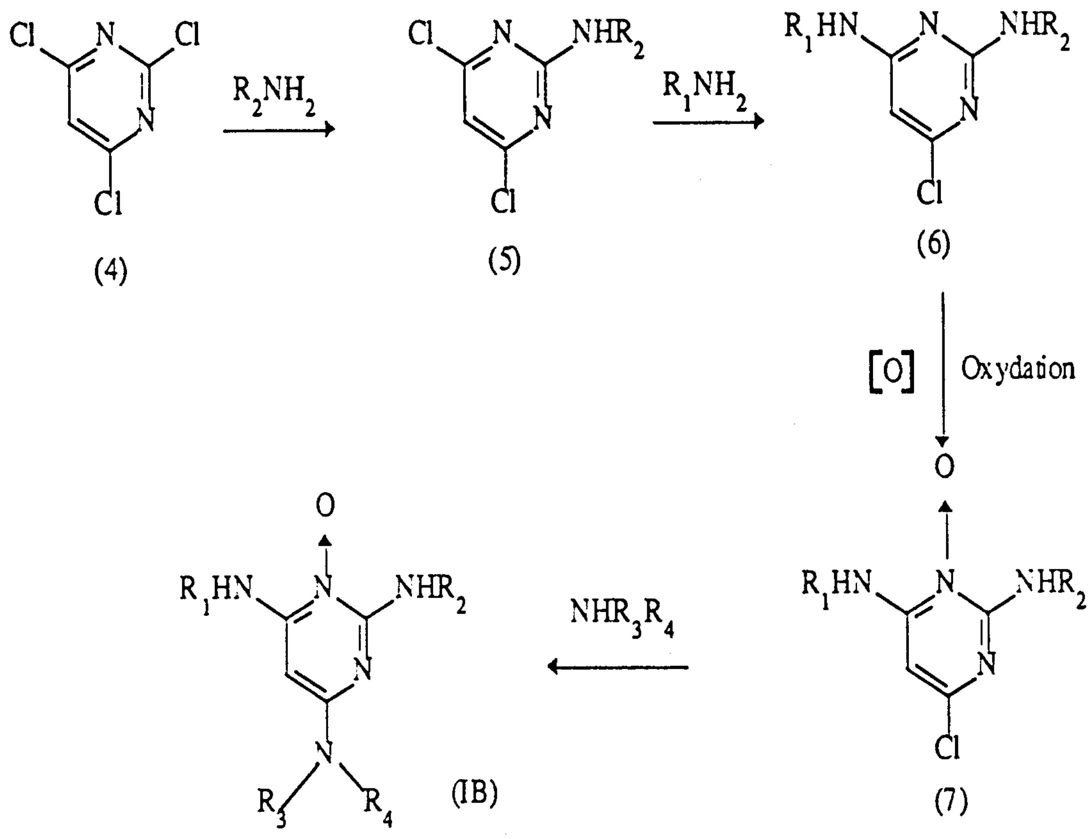


(4) (5) (6) (7) (IB)

SCHEMA B

Les composés particuliers de formule (IC) suivante :

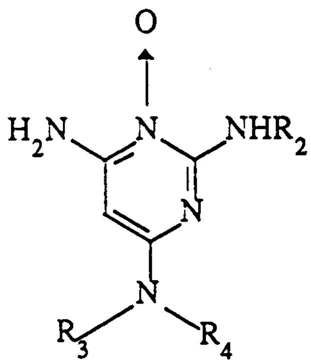

(IC)

dans laquelle $R_1$, $R_2$, $R_3$ et $R_4$ ont les mêmes significations indiquées ci-dessus, sont obtenus en faisant réagir de l'Oxone®($KHSO_5$) sur le thiométhyl-2 chloro-6 amino-4 pyrimidine de formule :

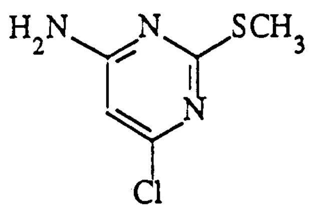

(8)

On introduit en position 2 du noyau pyrimidine du produit (9) obtenu un substituant amino $-NHR_2$. On effectue ensuite une oxydation en position 3 du noyau pyrimidine à l'aide d'un peracide carboxylique, de préférence l'acide metachloroperbenzoïque, suivie de l'introduction en position 6 de l'amine $NHR_3R_4$ par substitution nucléophile aromatique.

Ce procédé peut être représenté par le schéma suivant :

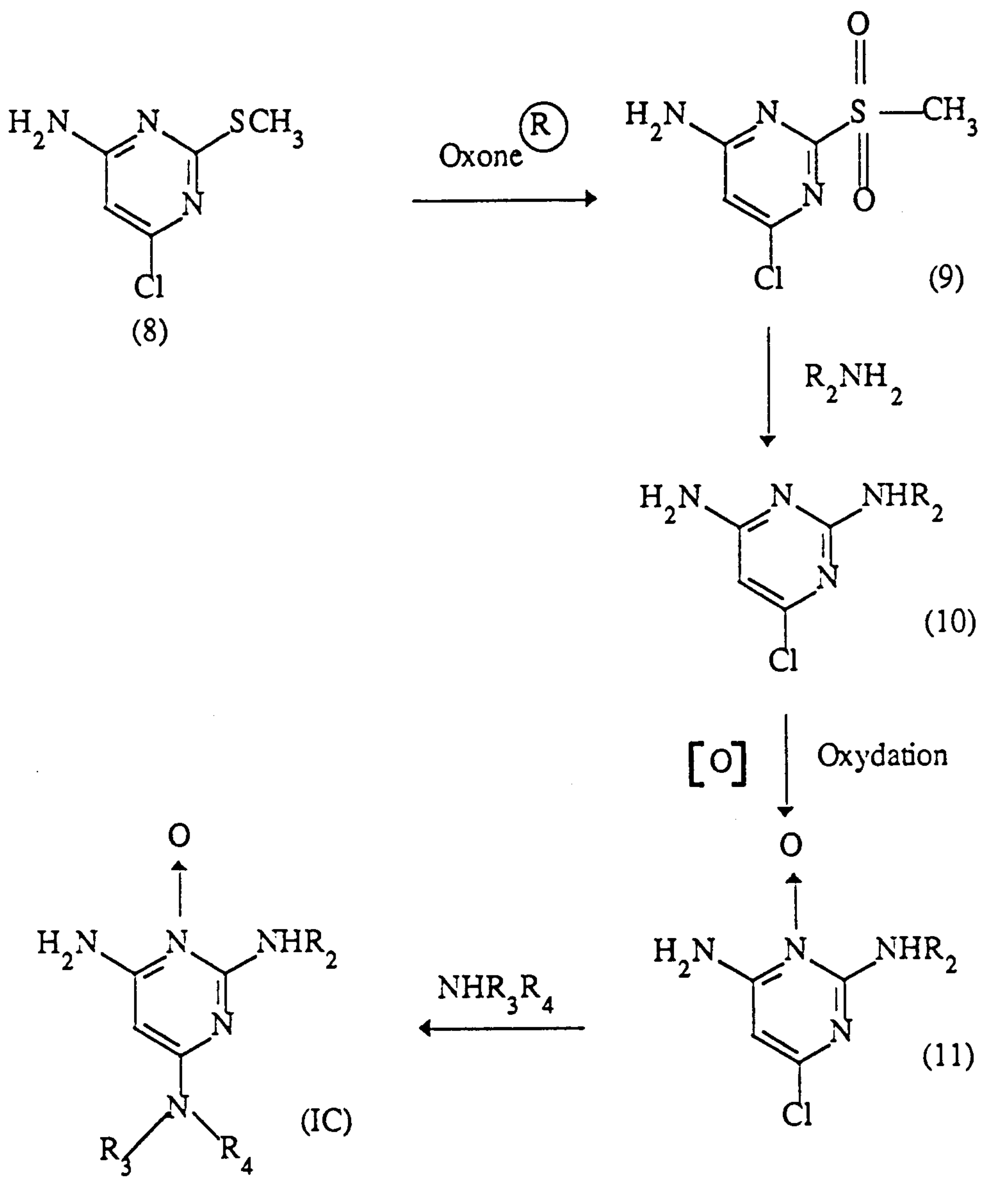


(8) (9) (10) (11) (IC)

SCHEMA C

Les composés particuliers de l'invention de formule générale (I) dans laquelle Y désigne un atome d'oxygène, peuvent être transformés en leurs homologues O-sulfates de formule (ID) définie ci-après par sulfatation chimique selon les méthodes classiques décrites dans la littérature (J. Med. Chem., 1983, 26, pages 1791-1793).

On utilise comme réactif de sulfatation les complexes de trioxyde de soufre-pyridine, de trioxyde de soufre-triéthylamine ou de trioxyde de soufre-éthyldiisopropylamine.

Les solvants utilisés sont de préférence le diméthylformamide, le chloroforme, l'acétonitrile ou leurs mélanges binaires. La température est de l'ordre de O à 25 °C et le temps de réaction varie de 1 à 24 heures.

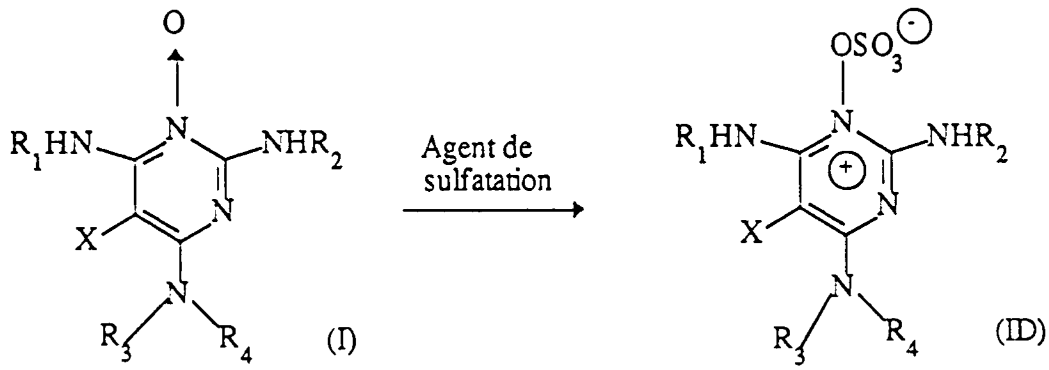


(I) (ID)

SCHEMA D

Les composés particuliers de formule suivante :

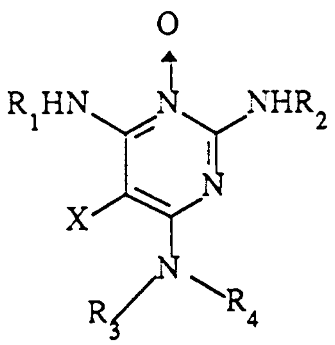

(IE)

dans laquelle $R_1$, $R_2$, $R_3$ et $R_4$ ont les mêmes significations indiquées ci-dessus et X désigne un halogène, sont obtenus à partir des composés (IA), (IB) et (IC) précédents par réaction avec un N-halogéno succinimide de formule :

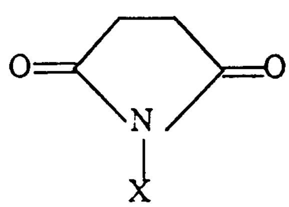

(12)

en présence d'un alcool tel que le méthanol.

Les compositions conformes à la présente invention, contenant dans un milieu physiologiquement acceptable, au moins un composé répondant à la formule (I) ou un de ses sels d'addition d'acides physiologiquement acceptables, peuvent être appliquées dans le domaine cosmétique ou pharmaceutique, notamment en application topique. Elles sont destinées pour le traitement et la prévention de la chute des cheveux et notamment de la pelade, de l'alopécie ainsi que des dermatites desquamantes.

Ces compositions peuvent comporter, à titre de milieu physiologiquement acceptable, tout milieu approprié pour l'application topique, soit en cosmétique, soit en pharmacie, et qui soit compatible avec la substance active.

Les composés conformes à l'invention peuvent se trouver dans ce milieu, soit à l'état dissous, soit à l'état dispersé, notamment sous forme micronisée.

Les compositions destinées à être utilisées en pharmacie se présentent sous forme d'onguent, de teinture, de crème, de pommade, de poudre, de timbre, de tampon imbibé, de solution, d'émulsion ou de

dispersion vésiculaire, de lotion, de gel, de spray ou de suspension. Elles peuvent être, soit anhydres, soit aqueuses, selon l'indication clinique.

Les composés selon l'invention sont présents dans ces compositions pharmaceutiques à des concentrations comprises entre 0,1 et 10% en poids, et en particulier comprises entre 0,2 et 5% en poids.

Les compositions cosmétiques sont notamment destinées à être utilisées sous forme de lotion, de gel, de savon, de shampooing, d'aérosol ou de mousse et contiennent, dans un support cosmétiquement acceptable, au moins un composé de formule (I) ou l'un de ses sels d'addition d'acides.

La concentration de ces composés de formule (I) dans ces compositions est, de préférence, comprise entre 0,01 et 5% en poids et en particulier entre 0,05 et 3% en poids.

Les compositions conformes à l'invention peuvent contenir différents additifs habituellement utilisés en cosmétique ou en pharmacie et en particulier des substances actives, telles que des agents hydratants comme la thiamorpholinone et ses dérivés ou l'urée; des agents antiséborrhéiques, tels que la S-carboxyméthylcystéine, la S-benzylcystéamine, et leurs dérivés; la thioxolone.

Les composés conformes à l'invention peuvent être associés à des composés améliorant encore leur activité sur la repousse et/ou sur le freinage de la chute des cheveux, tels que plus particulièrement les composés suivants :

- les esters d'acide nicotinique, dont plus particulièrement les nicotinates d'alkyle en $C_3$-$C_6$ et notamment le nicotinate de méthyle ou d'hexyle, le nicotinate de benzyle ou de tocophérol;
- les agents anti-inflammatoires stéroïdiens et non stéroïdiens bien connus dans l'état de la technique et en particulier l'hydrocortisone, ses sels et ses dérivés, l'acide niflumique;
- les rétinoïdes et plus particulièrement l'acide t-trans rétinoïque appelé encore trétinoïne, l'isotrétinoïne, le rétinol ou la vitamine A et ses dérivés, tels que l'acétate, le palmitate ou le propionate, le motrétinide, l'étrétinate, le t-trans rétinoate de zinc;
- les agents antibactériens choisis plus particulièrement parmi les macrolides, les pyranosides et les tétracyclines et notamment l'érythromycine;
- les agents antagonistes de calcium, tels que la Cinnarizine et le Diltiazem;
- des hormones, telles que l'estriol ou des analogues ou la thyroxine et ses sels;
- des agents antiandrogènes, tels que l'oxendolone, la spironolactone, le diéthylstilbestrol;
- des capteurs de radicaux OH, tels que le diméthylsulfoxyde;
- des oligosaccharides estérifiés, tels que ceux décrits dans EP-A-0 211 610 et EP-A-0 064 012;
- des dérivés d'acide hexosaccharique, tels que ceux décrits dans EP-A-0 375 388, en particulier l'acide glucosaccharique;
- des inhibiteurs de glycosidase, tels que ceux décrits dans EP-A-0 334 586, en particulier le D-glucaro 1,5-lactam;
- des inhibiteurs de glycosaminoglycanases et protéoglycanases, tels que ceux cités dans EP-A-0 277 428, en particulier la L-galactono 1,4-lactone;
- des inhibiteurs de tyrosine kinase, tels que ceux décrits dans EP-A-0 403 238, en particulier le 1-amido 1-cyano-(3,4-dihydroxyphényl)éthylène.

On peut également associer avec les composés de l'invention, éventuellement en mélange avec les autres, des composés tels que le Diazoxyde correspondant au méthyl-3 chloro-7[2H]benzothiadiazine-1,2,4 dioxyde-1,1; la Spiroxazone ou 7-(acétylthio)-4′,5′-dihydrospiro-[androst 4-ène-17,2′-(3′H)furan]-3 one; des phospholipides, tels que la lécithine; les acides linoléique et linolénique; l'acide salicylique et ses dérivés décrits plus particulièrement dans le brevet français n° 2 581 542, et plus particulièrement les dérivés d'acide salicylique porteurs d'un groupement alcanoyle ayant 2 à 12 atomes de carbone en position 5 du cycle benzénique; des acides hydroxycarboxyliques ou cétocarboxyliques et leurs esters, des lactones et leurs sels correspondants; l'anthraline, les caroténoïdes, les acides eicosatétranoïque-5,8,11,14 ou eicosatriynoïque-5,8,11, leurs esters et amides.

Les composés conformes à l'invention peuvent également être associés à des agents tensio-actifs dont ceux choisis parmi les agents tensio-actifs non-ioniques et amphotères.

Parmi les tensio-actifs non-ioniques, on citera les polyhydroxypropyléthers décrits notamment dans les brevets français n° 1.477.048; 2.091.516; 2.169.787; 2.328.763; 2.574.786; les alkyl($C_8$-$C_9$)phénols oxyéthylénés comportant de 1 à 100 moles d'oxyde d'éthylène et de préférence 5 à 35 moles d'oxyde d'éthylène; les alkylpolyglycosides de formule :

$$C_nH_{2n+1}(C_6H_{10}O_5)_xH \qquad (A)$$

dans laquelle n varie de 8 à 15 inclus et x de 1 à 10 inclus.

Parmi les agents tensio-actifs amphotères, on citera les amphocarboxyglycinates et les amphocarboxypropionates définis dans le dictionnaire CTFA, 3ème édition, 1982, et vendus, notamment, sous la dénomination MIRANOL® par la Société MIRANOL.

Les composés, selon l'invention, peuvent être introduits dans des supports qui améliorent encore l'activité au niveau de la repousse, en présentant à la fois des propriétés avantageuses sur le plan cosmétique, telles que des mélanges volatils ternaires d'alkyléthers d'alkylèneglycols, en particulier d'alkyle en $C_1$-$C_4$, d'alkylène en $C_1$-$C_4$ glycol ou de dialkylèneglycols, de préférence de dialkylène en $C_1$-$C_4$ glycol, d'alcool éthylique et d'eau, le solvant glycolique désignant les monoéthyléthers de l'éthylèneglycol, le monométhyléther du propylèneglycol, le monométhyléther du diéthylèneglycol.

Les composés conformes à l'invention peuvent également être introduits dans des supports physiologiquement acceptables gélifiés ou épaissis, tels que des supports essentiellement aqueux gélifiés par des hétérobiopolysaccharides, tels que la gomme de xanthane, les scléroglucanes, ou les dérivés de cellulose comme les éthers de cellulose, des supports hydroalcooliques gélifiés par des polyhydroxyéthylacrylates ou méthacrylates ou des supports essentiellement aqueux épaissis en particulier par des acides polyacryliques réticulés par un agent polyfonctionnel, tel que les CARBOPOL vendus par la Société GOODRICH.

Ces compositions peuvent également contenir des agents conservateurs, des agents stabilisants, des agents régulateurs de pH, des agents modificateurs de pression osmotique, des agents émulsifiants, des filtres UVA et UVB, des agents antioxydants, tels que l'$\alpha$-tocophérol, le butylhydroxyanisole, le butylhydroxytoluène.

Le milieu physiologiquement acceptable peut être constitué par de l'eau ou un mélange d'eau et d'un solvant ou un mélange de solvants, les solvants étant choisis parmi les solvants organiques acceptables sur le plan cosmétique ou pharmaceutique et choisis plus particulièrement parmi les alcools inférieurs en $C_1$-$C_4$, comme l'alcool éthylique, l'alcool isopropylique, l'alcool tertiobutylique, les alkylèneglycols et leurs alkyléthers cités ci-dessus.

Les solvants, lorsqu'ils sont présents, le sont dans des proportions comprises entre 1 et 80% en poids par rapport au poids total de la composition.

Les épaississants sont présents de préférence dans des proportions comprises entre 0,1 et 5% en poids et en particulier entre 0,4 et 3% en poids par rapport au poids total de la composition.

L'invention a également pour objet un procédé de traitement cosmétique des cheveux ou du cuir chevelu, consistant à leur appliquer au moins une composition telle que définie ci-dessus, en vue d'améliorer l'esthétique de la chevelure.

Un autre objet de l'invention est constitué par l'utilisation de la composition contenant les composés de formule (I) définie ci-dessus, pour la préparation d'un médicament ayant pour effet d'induire ou de stimuler la croissance des cheveux et de freiner leur chute.

Le traitement consiste principalement à appliquer sur les zones alopéciques du cuir chevelu d'un individu, la composition telle que définie ci-dessus.

Le mode d'application préféré consiste à appliquer 1 à 2 g de la composition sur la zone alopécique, à une fréquence de une à deux applications par jour, pendant 1 à 7 jours par semaine et ceci pendant une durée de 1 à 6 mois.

Les compositions peuvent notamment être utilisées dans le traitement de la pelade, de la chute des cheveux, des dermatites desquamantes ou de l'alopécie.

Les exemples suivants sont destinés à illustrer l'invention sans pour autant présenter un caractère limitatif.

EXEMPLES DE PREPARATION

EXEMPLE 1

**2-amino 4-propylamino 6-diméthylamino pyrimidine 3-oxyde.**

Le produit est préparé selon le schéma réactionnel :

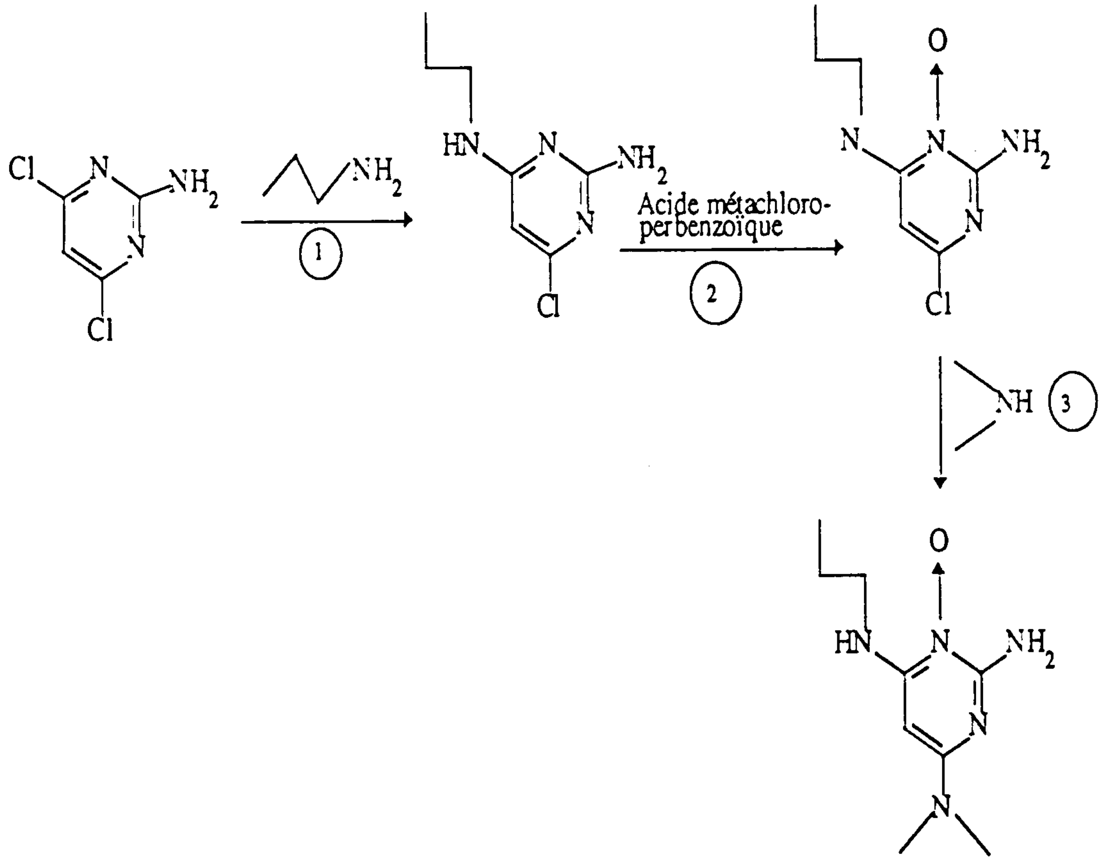


ETAPE 1

On disperse 10 g de 2-amino 4,6-dichloropyrimidine dans 100 ml de méthanol, puis on ajoute 10 ml de propylamine et on porte au reflux pendant 24 heures.

On concentre le milieu puis on ajoute 100 ml d'eau. Le précipité est filtré à froid puis recristallisé dans l'acétonitrile.

On sèche le solide blanc isolé pour obtenir 6,3 g de 2-amino 4-propylamino 6-chloropyrimidine.

Rendement = 55 %

P.F. = 104 °C.

Les spectres RMN $^{1}H$ et $^{13}C$ et de masse sont conformes à la structure attendue.

Analyse élémentaire pour $C_7H_{11}N_4Cl$

| | C | H | N | Cl |
|---|---|---|---|---|
| Calculé | 45,05 | 5,94 | 30,02 | 18,99 |
| Trouvé | 45,13 | 5,98 | 30,14 | 18,87 |

ETAPE 2

On ajoute 18 g d'acide metachloroperbenzoïque à une solution de 5 g de 2-amino 4-propylamino 6-chloropyrimidine dans 100 ml d'éthanol à O°C.

On agite ce mélange pendant 6 heures à O°C puis on filtre et on récupère 2,4 g de 2-amino 4-propylamino 6-chloropyrimidine 3-oxyde.

Rendement = 44%

P.F. = 190-195°C.

Les spectres RMN $^{1}H$ et $^{13}C$ et de masse sont conformes à la structure attendue.

ETAPE 3

On disperse 2 g de 2-amino 4-propylamino 6-chloropyrimidine 3-oxyde dans 50 ml de solution de diméthylamine à 33% dans l'éthanol.

On porte au reflux pendant 3 heures.

On évapore sous vide le milieu réactionnel.

L'huile obtenue est traitée par 20 ml d'éthanol chlorhydrique.

Le chlorhydrate obtenu est filtré et rincé à l'éther.

On traite le solide par la potasse alcoolique puis on filtre sur celite et on évapore sous vide. Le résidu est ensuite recristallisé dans 50 ml d'acétone plus 3 ml d'eau. On récupère 0,77 g de 2-amino 4-propylamino 6-diméthylamino pyrimidine 3-oxyde.

Rendement = 37%

P.F. = 217-218°C.

Les spectres RMN $^{1}H$ et $^{13}C$ et de masse sont conformes à la structure attendue.

Analyse élémentaire pour $C_9H_{17}N_5O$

| | C | H | N | O |
|---|---|---|---|---|
| Calculé | 51,17 | 8,11 | 33,15 | 7,57 |
| Trouvé | 51,24 | 8,15 | 33,23 | 7,70 |

EXEMPLE 2

**2-amino 4-isopropylamino 6-diméthylamino pyrimidine 3-oxyde.**

Le produit est préparé selon le schéma réactionnel :

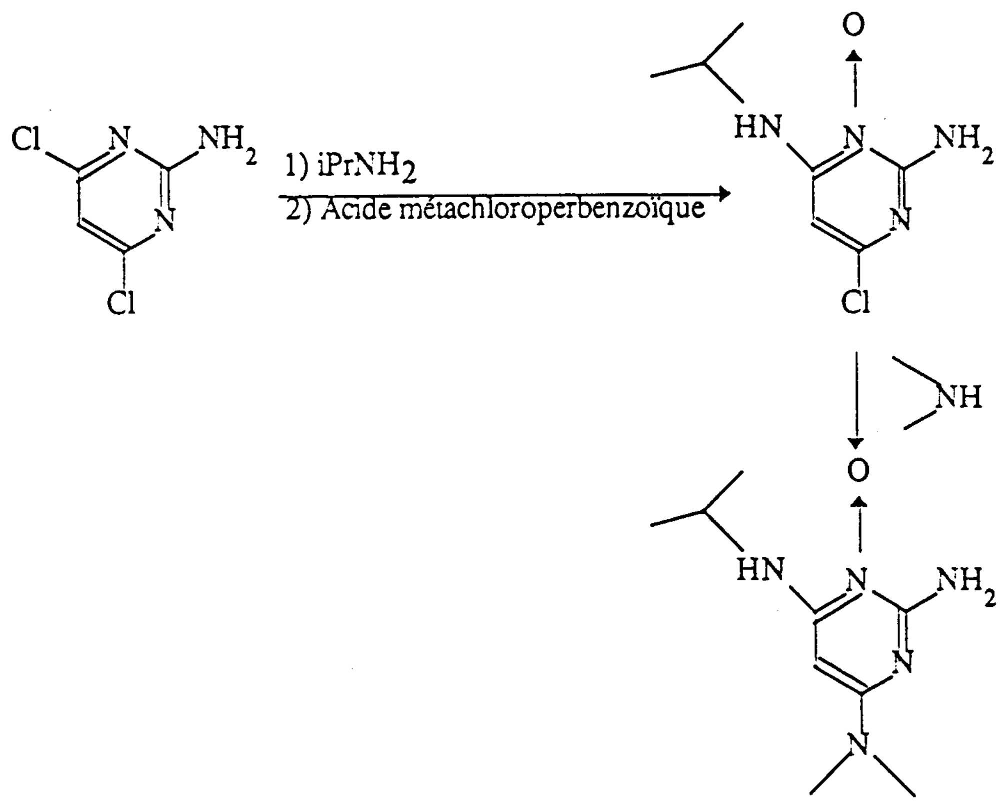


ETAPE 1

**2-amino 4-isopropylamino 6-chloro pyrimidine 3-oxyde.**

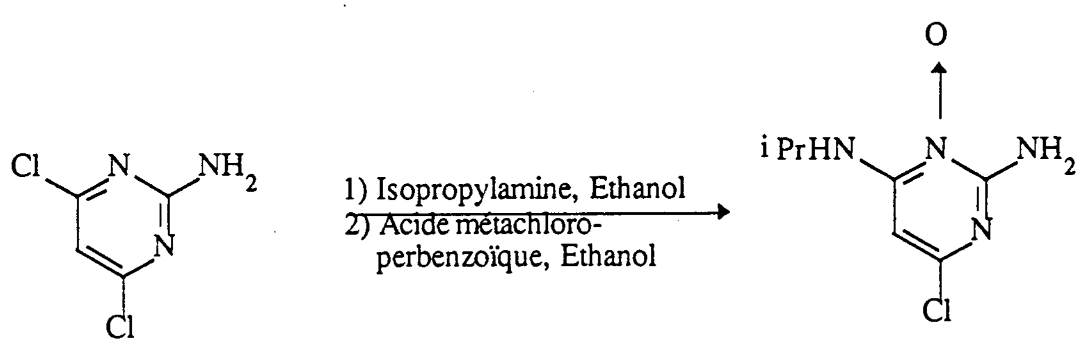


On place 10 g de 2-amino 4,6-dichloro pyrimidine en suspension dans 100 ml d'éthanol. On ajoute 18 g d'isopropylamine. Le milieu réactionnel est porté au reflux pendant 6 heures. On évapore à sec puis on reprend dans 50 ml d'eau. Après 1 heure d'agitation, le précipité est filtré sur verre fritté puis séché sous

vide sur anhydride phosphorique.

Le précipité obtenu est placé dans 300 ml d'éthanol. On additionne par petites spatules 28,70 g d'acide métachloroperbenzoïque. Le milieu réactionnel est agité 6 heures à température ambiante. On additionne 100 ml d'eau puis on évapore l'éthanol. Le pH du milieu réactionnel est alors ajusté à 1 par addition d'acide chlorhydrique concentré. Après 1 heure d'agitation, le précipité est filtré sur verre fritté, rincé par 50 ml d'eau et éliminé. Les filtrats sont amenés à pH=8 par addition de soude. On extrait par 2x100 ml de dichlorométhane. La phase organique est séchée sur sulfate de sodium, filtrée sur papier et évaporée à sec. Le précipité est repris dans 50 ml d'éther éthylique, agité 1 heure, filtré sur verre fritté. On obtient 4,05 g de 2-amino 4-isopropylamino 6-chloro pyrimidine 3-oxyde.

Rendement = 33%

Les spectres RMN $^1H$ et de masse sont conformes à la structure attendue.

ETAPE 2

**2-amino 4-isopropylamino 6-diméthylamino pyrimidine 3-oxyde.**

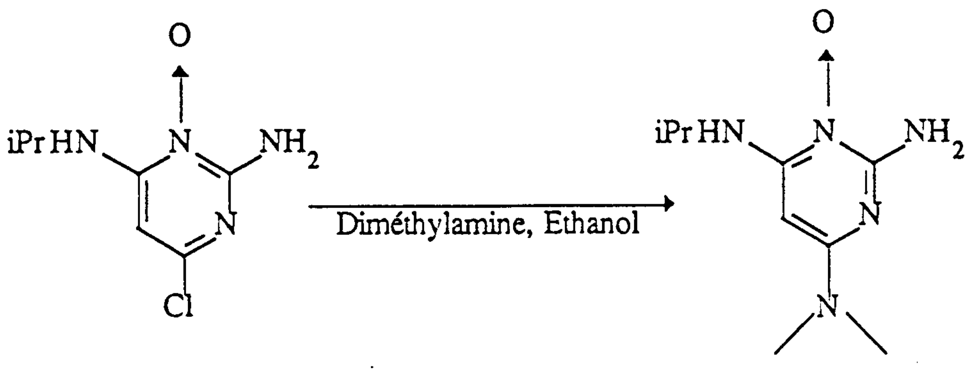


On place 4,5 g de 2-amino 4-isopropylamino 6-chloro pyrimidine 3-oxyde dans 25 ml d'éthanol. On additionne 20 ml de diméthylamine en solution à 33% dans l'éthanol. Le milieu réactionnel est porté au reflux 2 h 30 minutes puis évaporé à sec. Le précipité est solubilisé dans 10 ml d'éthanol. On additionne de l'éthanol chlorhydrique jusqu'à pH=1. On agite 1 heure puis on additionne 20 ml d'éther éthylique. Le précipité est filtré sur verre fritté, rincé par 20 ml d'éther éthylique, séché sous vide.

Le précipité est repris dans 20 ml d'eau. On ajuste le pH à 8 par addition de soude. Après 1 heure d'agitation, le précipité est filtré sur verre fritté. On le recristallise dans 55 ml d'acétonitrile-eau (9/1). On obtient 2,55 g de 2-amino 4-isopropylamino 6-diméthylamino pyrimidine 3-oxyde.

Rendement = 56%

Analyse élémentaire pour $C_9H_{17}N_5O$ ; M = 211

| | C | H | N | O |
|---|---|---|---|---|
| Calculé | 51,18 | 8,06 | 33,17 | 7,58 |
| Trouvé | 51,15 | 8,07 | 33,11 | 7,74 |

Les spectres RMN $^1H$ et de masse sont conformes à la structure attendue.

EXEMPLE 3

**2,4-N,N'-dipropylamino 6-diméthylamino pyrimidine 3-oxyde.**

Le produit est préparé selon le schéma réactionnel suivant :

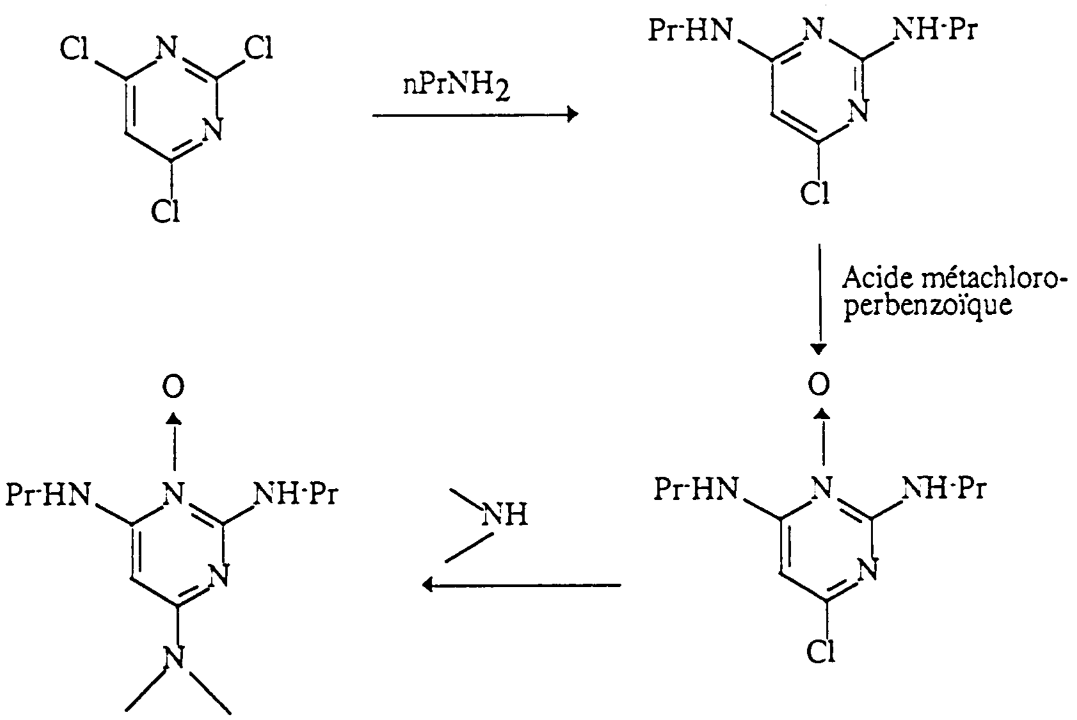


ETAPE 1

**2,4-N,N'-dipropylamino 6-chloro pyrimidine.**

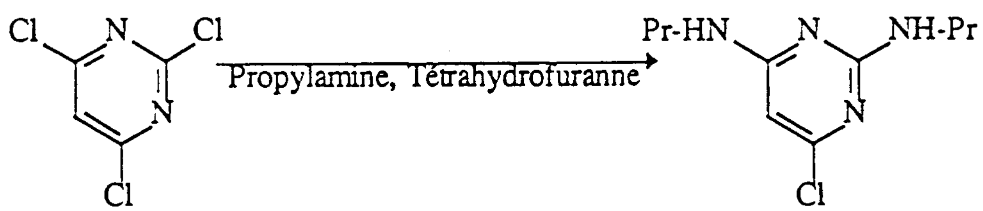


On place 10 g de 2,4,6-trichloro pyrimidine dans 100 ml de tétrahydrofuranne. On ajoute, à température ambiante, 19,20 g de propylamine en solution dans 20 ml de tétrahydrofuranne. Le milieu réactionnel est porté au reflux pendant 21 heures. On revient à température ambiante puis on lave par 100 ml d'eau. La phase aqueuse est extraite par 10 ml de dichlorométhane. Les phases organiques sont jointes, séchées sur sulfate de sodium, filtrées sur papier et évaporées à sec. L'huile obtenue est purifiée par chromatographie sur colonne de silice (éluant : dichlorométhane). On obtient 10,75 g de 2,4-N,N'-dipropylamino 6-chloro pyrimidine.

Rendement = 86%

Analyse élémentaire pour $C_{10}H_{17}N_4Cl$ ; M = 228,5

| | C | H | N | Cl |
|---|---|---|---|---|
| Calculé | 52,51 | 7,44 | 24,50 | 15,54 |
| Trouvé | 52,53 | 7,40 | 24,60 | 15,54 |

Les spectres RMN $^1H$ et de masse sont conformes à la structure attendue.

ETAPE 2

**2,4-N,N'-dipropylamino 6-chloro pyrimidine 3-oxyde.**

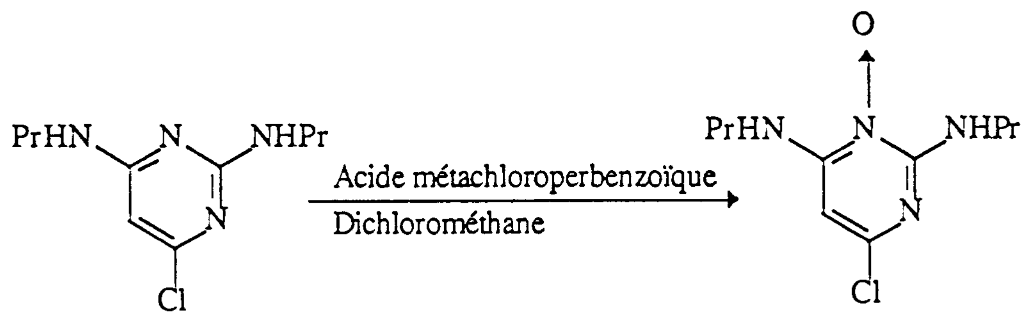


On place 6,35 g de 2,4-N,N'-dipropylamino 6-chloro pyrimidine dans 200 ml de dichlorométhane. On ajoute 21,80 g d'acide métachloroperbenzoïque par petites spatules. Le milieu réactionnel est agité à température ambiante pendant 4 heures. On évapore à sec. Le résidu est repris dans 200 ml d'eau. On ajuste le pH à 8 par addition de soude. On extrait par 3x100 ml de dichlorométhane. La phase organique est séchée sur sulfate de sodium, filtrée sur papier, évaporée à sec. L'huile obtenue est purifiée par chromatographie sur colonne de silice (Eluants : acétate d'éthyle/méthanol avec un gradient de polarité de 0 à 5% de méthanol). On obtient 620 mg de 2,4-N,N'-dipropylamino 6-chloro pyrimidine 3-oxyde.

Rendement: 9 %

Les spectres RMN $^1H$ et de masse sont conformes à la structure attendue.

ETAPE 3

**2,4-N,N'-dipropylamino 6-diméthylamino pyrimidine 3-oxyde.**

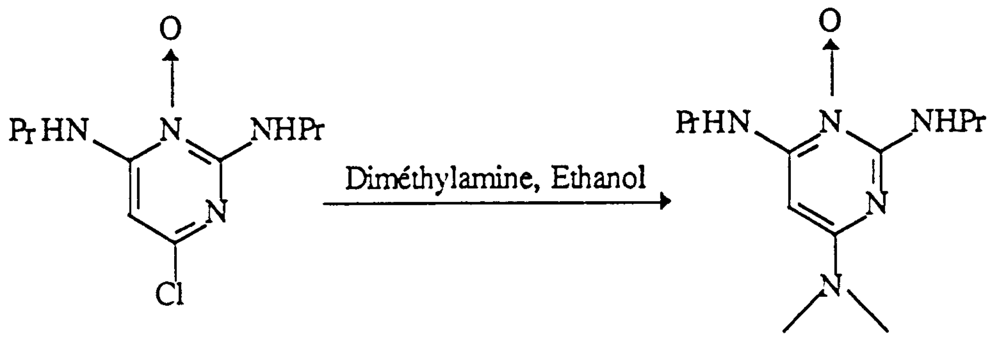


On place 620 mg de 2,4-N,N'-dipropylamino 6-chloro pyrimidine 3-oxyde dans 15 ml d'éthanol. On additionne 1 ml de diméthylamine en solution à 33% dans l'éthanol. Le milieu réactionnel est porté au reflux pendant 7 h 30 minutes puis évaporé à sec. On reprend dans 10 ml d'éther éthylique. On additionne de l'éthanol chlorhydrique jusqu'à pH acide. Après 1 heure d'agitation, on filtre sur verre fritté. Le précipité est repris dans 5 ml d'eau. On ajuste le pH à 8 par addition de soude. Après 1 heure d'agitation, on filtre sur verre fritté. Le précipité est recristallisé dans 3 ml d'acétone. On obtient 120 mg de 2,4-N,N'-dipropylamino 6-diméthylamino pyrimidine 3-oxyde.

Rendement = 19 %

Analyse élémentaire pour $C_{12}H_{23}N_5O$ ; M = 253

| | C | H | N | O |
|---|---|---|---|---|
| Calculé | 56,92 | 9,09 | 27,67 | 6,32 |
| Trouvé | 57,04 | 9,13 | 27,85 | 6,38 |

Les spectres RMN $^1H$ et de masse sont conformes à la structure attendue.

EXEMPLE 4

**Synthèse du 2-propylamino 4-amino 6-N,N-diméthylamino 6-pyrimidine 3-oxyde.**

ETAPE 1

***2-méthylsulfonyl 4-amino 6-chloro pyrimidine.***

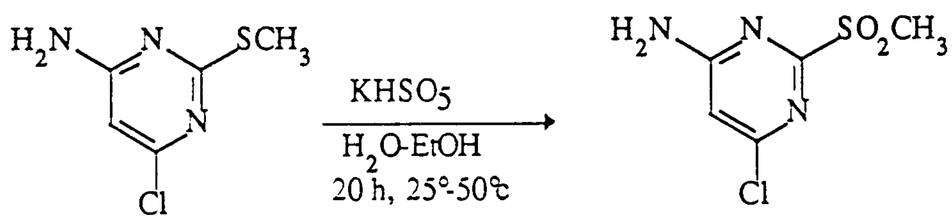


Mode opératoire :

Dans un ballon tricol de 1 litre, on introduit 13,17 g de 2-thiométhyl 4-amino 6-chloro pyrimidine en solution dans 400 ml d'éthanol. On refroidit cette solution vers + 4 ° C, puis on additionne 69,14 g d'oxone® en solution dans 300 ml d'eau. Le milieu réactionnel devient hétérogène. On l'agite fortement. Après 16 heures de réaction à 25 ° C, on chauffe légèrement la réaction vers 50 ° C pendant encore 4 heures. Le milieu est ensuite refroidi entre 5 ° et 10 ° C, puis filtré. On reprend le précipité obtenu sous agitation dans 300 ml d'eau une première fois, puis on le filtre. On le reprend comme précédemment dans 100 ml d'eau. Après filtration et essorage, le produit est séché sous vide et sur $P_2O_5$ à 70 ° C pendant une nuit. On obtient 10,85 g de 2-méthylsulfonyl 4-amino 6-chloro pyrimidine sous forme d'une poudre blanche.

Rendement = 83%

P.F. = 239 ° C.

Analyse élémentaire pour $C_5H_6Cl\,N_3O_2S$ ; M = 207,64

| | C | H | Cl | N | O | S |
|---|---|---|---|---|---|---|
| Calculé | 28,92 | 2,89 | 17,11 | 20,24 | 15,42 | 15,42 |
| Trouvé | 29,10 | 2,95 | 16,98 | 20,12 | 15,59 | 15,40 |

Les spectres RMN $^1H$ et de masse sont conformes à la structure attendue.

ETAPE 2

*2-n-propylamine 4-amino 6-chloro pyrimidine*

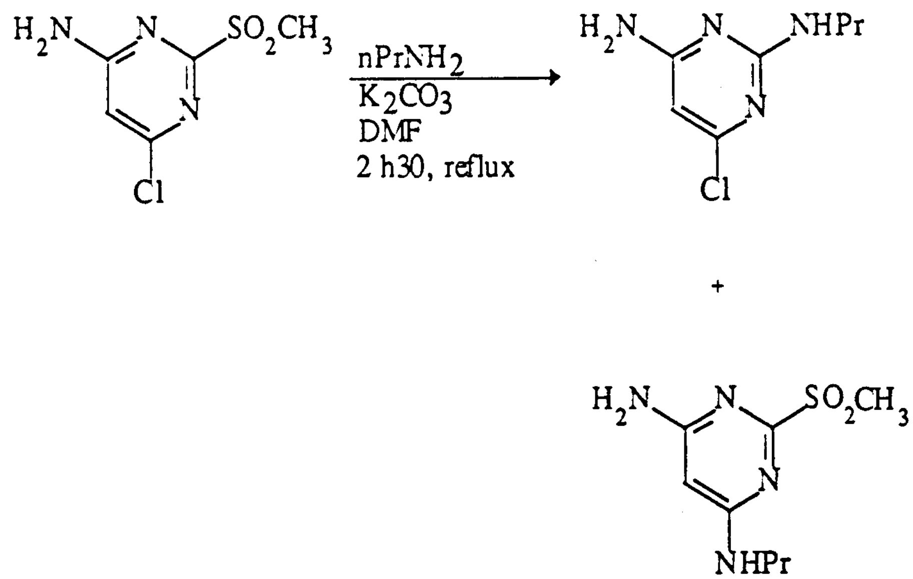


Dans un tricol de 250 ml équipé d'un thermomètre et d'un réfrigérant ascendant, on introduit 6,04 g de 2-méthylsulfonyl 4-amino 6-chloro pyrimidine dans 150 ml de DMF, puis 2,56 g de n-propyl-amine et 3 g de $K_2CO_3$. On porte la réaction au reflux du DMF pendant 2 h 30 minutes.

Le solvant est évaporé sous vide et le brut obtenu est repris dans 100 ml d'eau. Cette phase aqueuse est extraite avec 5x50 ml d'éther diéthylique. Les phases éthérées sont réunies, séchées sur $Na_2SO_4$ et le solvant est évaporé. On recueille 5,4 g de produit brut liquide très visqueux. Ce produit brut est chromatographié sur colonne de silice [éluant : $CH_2Cl_2/CH_3OH(98/2)$]. On obtient une première fraction de 2,61 g de 2-propylamino 4-amino 6-chloropyrimidine (rendement = 48%; P.F. = 92-94 °C en capillaire), et une deuxième fraction de 1,2 g de 2-méthylsulfonyl 4-amino 6-propylamino pyrimidine (rendement = 18%; P.F. = 100-102 °C).

**2-propylamino 4-amino 6-chloro pyrimidine**

Analyse élémentaire pour $C_7H_{11}N_4Cl$ ; M = 186,64

| | C | H | N | Cl |
|---|---|---|---|---|
| Calculé | 45,04 | 5,95 | 30,02 | 18,99 |
| Trouvé | 44,95 | 6,02 | 30,00 | 18,89 |

Les spectres RMN $^1H$ et de masse sont conformes à la structure attendue.

2-méthylsulfonyl 4-amino 6-propylamino pyrimidine

Analyse élémentaire pour $C_8H_{14}N_4O_2S$ ; M = 230,29

| | C | H | N | O | S |
|---|---|---|---|---|---|
| Calculé | 41,73 | 6,13 | 24,33 | 13,90 | 13,92 |
| Trouvé | 41,73 | 6,13 | 24,32 | 14,06 | 14,16 |

Les spectres RMN $^{1}H$ et de masse sont conformes à la stucture attendue.

ETAPE 3

***2-n-propylamino 4-amino 6-chloro pyrimidine 3-oxyde***

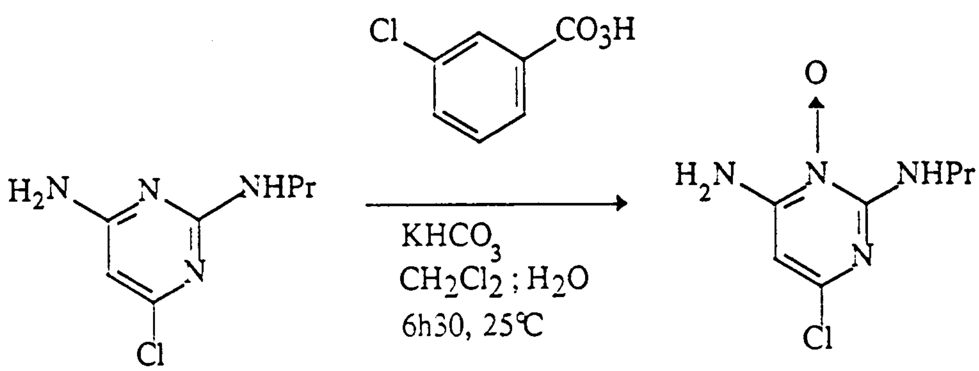


Mode opératoire :

Dans un tricol de 100 ml, on introduit 1,56 g de $KHCO_3$ en solution dans 20 ml d'eau et 1,22 g de 2-n-propylamino 4-amino 6-chloro pyrimidine en solution dans 20 ml de dichlorométhane. On refroidit le milieu biphasique vers + 10°C et on additionne par portions 4,1 g d'acide m-chloroperbenzoïque à 55%. On laisse le milieu revenir à température ambiante. Après 6 h 30 minutes de réaction, on évapore le dichlorométhane et on acidifie la phase aqueuse avec 1,83 ml d'acide chlorhydrique à 37%. On filtre l'acide m-chlorobenzoïque qui précipite puis on alcalinise le filtrat avec 2,21 ml de soude 10N. On extrait cette phase alcaline avec 4x50 ml de butanol.

On réunit les phases butanoliques et on évapore le solvant. On obtient un liquide visqueux que l'on cristallise dans 25 ml d'éther diéthylique. Après filtration, on recueille 0,83 g de 2-n-propylamino 4-amino 6-chloro pyrimidine 3-oxyde brut.

Rendement brut = 63 %

Les spectres RMN $^{1}H$ et de masse sont conformes à la structure attendue.

ETAPE 4

***2-n-propylamino 4-amino 6-N,N-diméthylamino pyrimidine 3-oxyde***

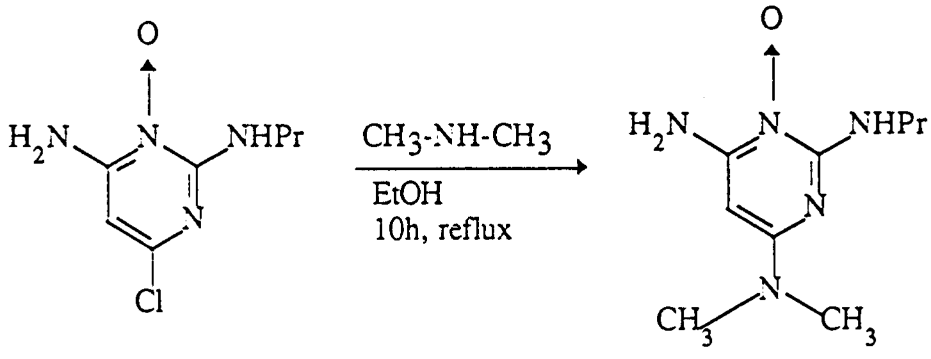


Mode opératoire :

Dans un ballon de 100 ml, on introduit 0,39 g de 2-n-propylamino 4-amino 6-chloro pyrimidine 3-oxyde en solution dans 20 ml d'éthanol et 17 ml d'une solution éthanolique de N,N-diméthylamine à 33%. On porte le milieu au reflux pendant 10 heures. On évapore le solvant et on chromatographie sur colonne de silice le brut obtenu (éluant : $CH_2Cl_2/CH_3OH$ = 92/8). On recueille 0,25 g de 2-n-propylamino 4-amino 6-N,N-diméthylamino pyrimidine 3-oxyde.

Rendement = 62%

Les spectres RMN $^1H$ et de masse sont conformes à la structure attendue.

EXEMPLE 5

**Synthèse du 2-amino 4-méthylamino 6-pyrrolidino pyrimidine 3-oxyde**

ETAPE 1

***2-amino 4-méthylamino 6-chloro pyrimidine***

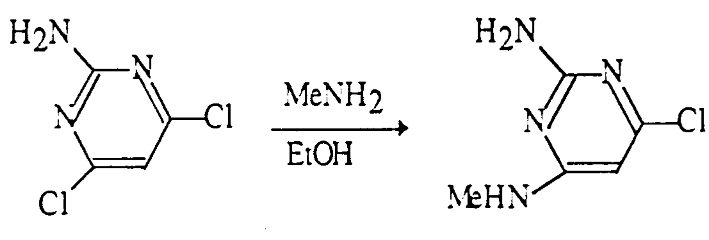


On place 10 g de 2-amino 4,6-dichloropyrimidine en suspension dans 100 ml d'éthanol. On additionne 11,80 g de méthylamine en solution à 40% dans l'eau. Le milieu réactionnel est porté au reflux pendant 3 heures. Après être revenu à température ambiante, on additionne 4 g d'hydroxyde de potassium en solution dans 40 ml d'éthanol. Après 1/2 heure d'agitation, le milieu réactionnel est filtré sur papier. On évapore à sec. Le précipité obtenu est repris dans 25 ml d'eau, filtré sur verre fritté, rincé par 25 ml d'eau puis séché sous vide et sur anhydride phosphorique. On obtient 8,50 g de 2-amino 4-méthyl amino 6-chloro pyrimidine.

Rendement = 88%

ANALYSES :

- Spectre de masse conforme
- Spectre RMN $^1H$ conforme.

ETAPE 2

***2-amino 4-méthylamino 6-chloro pyrimidine 3-oxyde***

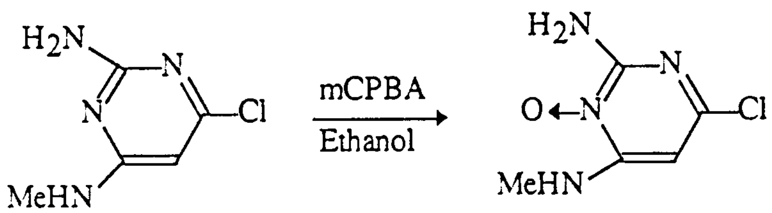


On place 4 g de 2-amino 4-méthylamino 6-chloro pyrimidine en suspension dans 50 ml d'éthanol. Après avoir refroidi à 10°C, on ajoute goutte à goutte 11,9 g d'acide métachloroperbenzoïque en solution dans 100 ml d'éthanol. En fin d'addition, on revient à température ambiante et on agite 3 heures. Le milieu réactionnel est refroidi à 5°C, puis filtré sur verre fritté. Le précipité obtenu est recristallisé dans 160 ml d'un mélange éthanol-eau 2/3-1/3. On obtient 1,60 g de 2-amino 4-méthylamino 6-chloro pyrimidine 3-oxyde.

Rendement = 36%

ANALYSES :

- Spectre RMN $^1H$ conforme
- Spectre de masse conforme

ETAPE 3

***2-amino 4-méthylamino 6-pyrrolidino pyrimidine 3-oxyde***

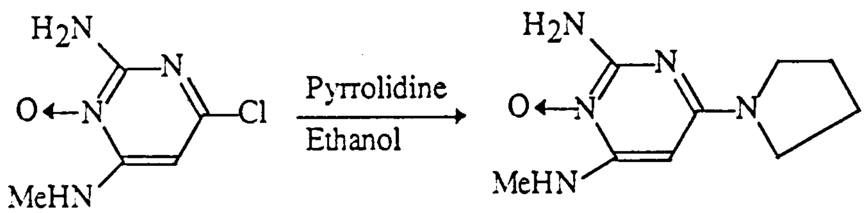


On place 3,4 g de 2-amino 4-méthylamino 6-chloro pyrimidine 3-oxyde en suspension dans 70 ml d'éthanol. On ajoute 4,15 g de pyrrolidine. Le milieu réactionnel est porté au reflux pendant 7 heures. On revient à température ambiante puis on ajoute 1,30 g d'hydroxyde de potassium à 85% en solution dans 15 ml d'éthanol. Après 1heure d'agitation, on filtre sur papier. Les filtrats sont évaporés à sec. Le résidu obtenu est recristallisé dans 50 ml d'un mélange acétonitrile-eau 9/1. On obtient 2,40 g de 2-amino 4-méthylamino 6-pyrrolidino pyrimidine 3-oxyde.

Rendement = 56%

ANALYSES :

- Spectre RMN $^1$H conforme
- Spectre de masse conforme

Analyse élémentaire pour $C_9H_{15}N_5O$ ; M = 209

| | C | H | N | O |
|---|---|---|---|---|
| Calculé avec 0,5 mole d'eau | 49,54 | 7,34 | 32,11 | 11,01 |
| Trouvé | 49,54 | 7,22 | 32,15 | 11,30 |

EXEMPLE 6

**Synthèse du 2-amino 4-tertiobutylamino 6-diméthylamino pyrimidine 3-oxyde**

ETAPE 1

***2-amino 4-tert-butylamino 6-chloro pyrimidine***

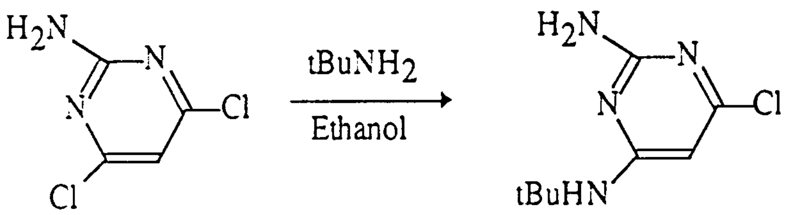


On place 10 g de 2-amino 4,6-dichloro pyrimidine en suspension dans 100 ml d'éthanol. On ajoute 44,50 g de tert-butylamine. Le milieu réactionnel est porté au reflux 48 heures. On évapore à sec. Le résidu est repris dans 50 ml d'eau. On agite 1 heure puis on filtre le précipité sur verre fritté. On recristallise dans 60 ml d'un mélange éthanol-eau 50/50. On obtient 9,25 g de 2-amino 4-tert-butylamino 6-chloro pyrimidine.

Rendement = 75 %

ANALYSES :

- Spectre RMN $^1$H conforme
- Spectre de masse conforme

ETAPE 2

***2-amino 4-tert-butylamino 6-chloro pyrimidine 3-oxyde***

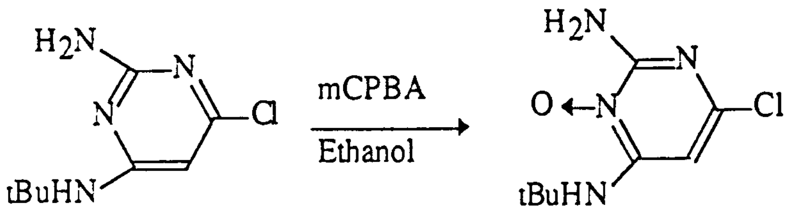


On place 9 g de 2-amino 4-tert-butylamino 6-chloro pyrimidine en suspension dans 90 ml d'éthanol. On additionne goutte à goutte 24,70 g d'acide métachloroperbenzoïque en solution dans 250 ml d'éthanol. Le milieu réactionnel est agité 7 heures 30 minutes à température ambiante. On évapore à sec. Le résidu est repris dans 100 ml d'eau. Le pH est ajusté à 1 par addition d'acide chlorhydrique concentré. Après 1 heure d'agitation, le précipité est filtré sur verre fritté puis éliminé. Les filtrats sont amenés à pH 8 par addition d'hydroxyde de sodium concentré puis extraits par 3 x 100 ml de dichlorométhane. La phase organique est séparée, séchée sur sulfate de sodium, filtrée sur papier et évaporée à sec. Le résidu obtenu est recristallisé dans 60 ml d'acétonitrile. On obtient 1,30 g de 2-amino 4-tert-butylamino 6-chloro pyrimidine 3-oxyde.

Rendement = 14%

ANALYSES :

- Spectre RMN $^1$H conforme
- Spectre de masse conforme

ETAPE 3

***2-amino4-tert-butylamino 6-diméthylamino pyrimidine 3-oxyde***

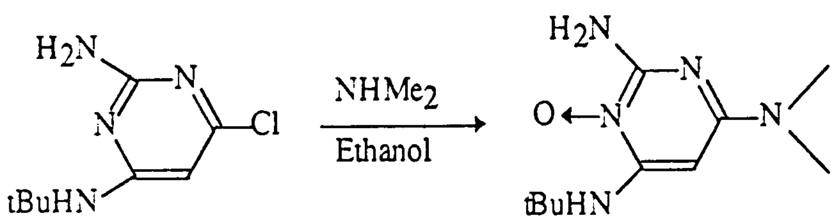


On place 1,25 g de 2-amino 4-tert-butylamino 6-chloro pyrimidine 3-oxyde en suspension dans 15 ml d'éthanol. On ajoute 5 ml de diméthylamine en solution à 33% dans l'éthanol. Le milieu réactionnel est porté au reflux 3 heures puis évaporé à sec. Le résidu est repris dans 10 ml d'eau. Le pH est ajusté à 8 par addition d'hydroxyde de sodium concentré. On extrait par 3 x 20 ml de dichlorométhane. La phase organique est séchée sur sulfate de sodium, filtrée sur papier, évaporée à sec. Le précipité obtenu est recristallisé dans 10 ml d'acétonitrile. On obtient 520 mg de 2-amino 4-tert-butylamino 6-diméthylamino pyrimidine 3-oxyde.

Rendement = 40%

ANALYSES :

- Spectre RMN $^1$H conforme
- Spectre de masse conforme

Analyse élémentaire pour $C_{10}H_{19}N_5O$ ; M = 225

| | C | H | N | O |
|---|---|---|---|---|
| Calculé | 53,33 | 8,45 | 31,11 | 7,11 |
| Trouvé | 53,35 | 8,52 | 31,22 | 7,17 |

EXEMPLE 7

**Synthèse du 2,4-N,N'-diéthylamino 6-diméthylamino pyrimidine 3-oxyde**

ETAPE 1

***2,4-N,N-diéthylamino 6-chloro pyrimidine***

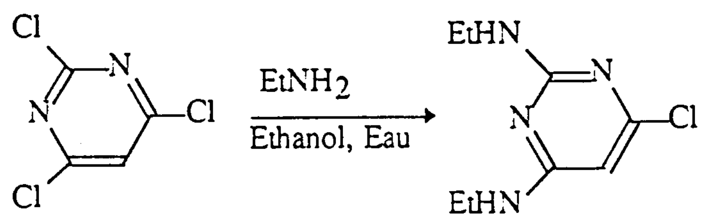


On place 15 g de 2,4,6-trichloro pyrimidine en solution dans 150 ml d'éthanol. On ajoute goutte à goutte 78 g d'éthylamine en solution à 33% dans l'eau. Le milieu réactionnel est porté à reflux pendant 7 heures. On évapore l'éthanol. Le résidu est extrait par 3 x 100 ml de dichlorométhane. La phase organique est lavée par 100 ml d'eau, séchée sur sulfate de sodium, évaporée à sec. On obtient 15,90 g de 2,4-N,N'-diéthylamino pyrimidine.

Rendement = 97%

ANALYSES :

- Spectre RMN $^1$H conforme
- Spectre de masse conforme

Analyse élémentaire pour $C_8H_{13}N_4Cl$ ; M = 200,5

| | C | H | N | Cl |
|---|---|---|---|---|
| Calculé | 47,88 | 6,48 | 27,93 | 17,70 |
| Trouvé | 47,76 | 6,57 | 27,69 | 17,90 |

ETAPE 2

***2,4-N,N-diéthylamino 6-chloro pyrimidine 3-oxyde***

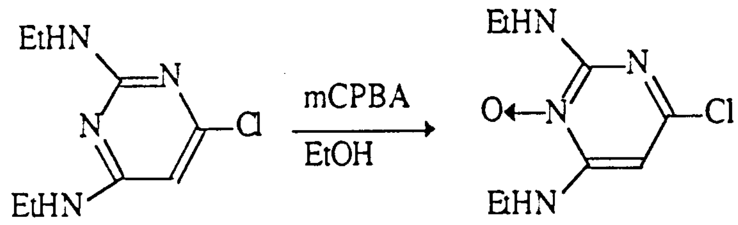


On solubilise 12 g de 2,4-N,N'-diéthylamino 6-chloropyrimidine dans 50 ml d'éthanol. Le milieu réactionnel est refroidi à 10 °C. On additionne goutte à goutte 32,9 g d'acide métachloroperbenzoïque en solution dans 180 ml d'éthanol. Après 24 heures d'agitation à température ambiante, on additionne 200 ml d'eau. On évapore l'éthanol. Le pH de la phase aqueuse résultante est ajusté à 1 par addition d'acide chlorhydrique concentré. Le milieu réactionnel est agité 1 heure, filtré sur verre fritté, rincé par 50 ml d'eau. On élimine le précipité. Les filtrats sont amenés à pH 8 par addition de soude puis extraits par 3 x 100 ml de dichlorométhane. La phase organique est séchée sur sulfate de sodium et évaporée à sec. Le résidu est purifié par chromatographie sur colonne de silice (éluant : acétate d'éthyle/méthanol). On obtient 2,2 g de 2,4-N,N'-diéthylamino 6-chloro pyrimidine 3-oxyde.

Rendement = 17%

ANALYSES :

- Spectre de masse conforme
- Spectre RMN $^1H$ conforme

ETAPE 3

***2,4-N,N-diéthylamino 6-diméthylamino pyrimidine 3-oxyde***

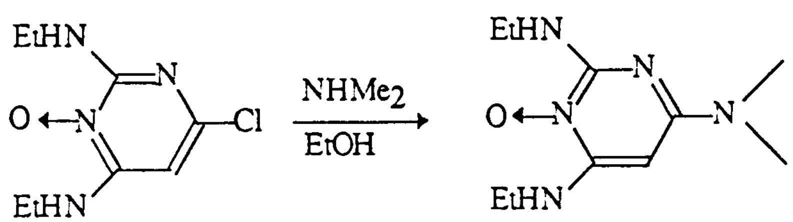


On place 2,20 g de 2,4-N,N'-diéthylamino 6-chloro pyrimidine 3-oxyde en solution dans 20 ml d'éthanol. On ajoute 10 ml de dimétylamine en solution à 33% dans l'éthanol. Le milieu réactionnel est porté au reflux pendant 5 heures puis évaporé à sec. On reprend le résidu dans 25 ml d'eau. On ajuste le pH à 8 par addition de soude concentrée puis on extrait par 4 x 25 ml d'acétate d'éthyle. La phase organique est séchée sur sulfate de sodium, filtrée sur papier et évaporée à sec. On recristallise dans 40 ml d'éther isopropylique. On obtient 750 mg de 2,4-N,N'-diéthylamino 6-diméthylamino pyrimidine 3-oxyde.

Rendement = 33%

ANALYSES :

- Spectre de masse conforme
- Spectre RMN $^1H$ conforme

Analyse élémentaire pour $C_{10}H_{19}N_5O$ ; M = 225

| | C | H | N | O |
|---|---|---|---|---|
| Calculé | 53,33 | 8,44 | 31,11 | 7,11 |
| Trouvé | 53,20 | 8,44 | 31,13 | 7,24 |

EXEMPLE 8

**Synthèse du 2-amino 4-propylamino 6-pipéridino pyrimidine 3-oxyde**

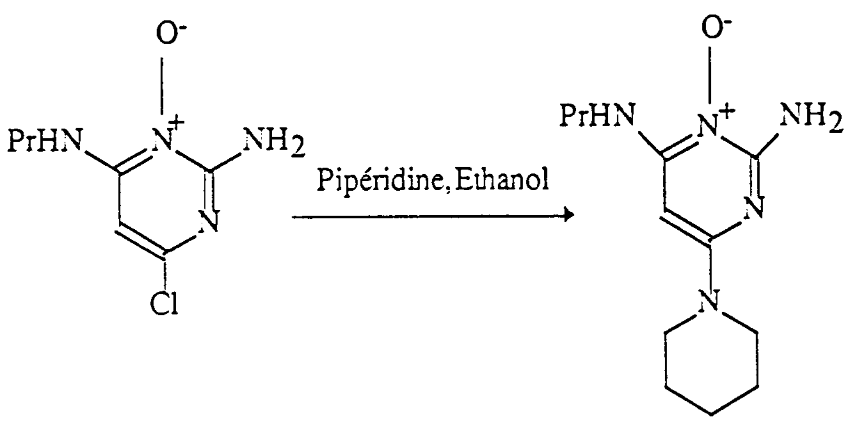


On place 2 g de 2-amino 4-propylamino 6-chloro pyrimidine 3-oxyde, préparé selon les deux premières étapes du procédé de l'exemple 1, en suspension dans 20 ml d'éthanol. On additionne 1,75 g de pipéridine. Le milieu réactionnel est porté au reflux 19 heures. On revient à température ambiante, puis on additionne 660 mg d'hydroxyde de potassium en solution dans 10 ml d'éthanol. Après 1 heure d'agitation, on filtre sur papier. Les filtrats sont évaporés à sec. Le résidu est recristallisé dans 25 ml d'acétone. On obtient 950 mg de 2-amino 4-propylamino 6-pipéridino pyrimidine 3-oxyde.

Rendement = 38%

ANALYSES :

- Spectre de masse conforme
- Spectre RMN $^1$H conforme

Analyse élémentaire pour $C_{12}H_{21}N_5O$ ; M = 251

| | C | H | N | O |
|---|---|---|---|---|
| Calculé | 57,37 | 8,37 | 27,89 | 6,37 |
| Trouvé | 57,44 | 8,42 | 27,88 | 6,21 |

EXEMPLE 9

**Synthèse du 2-amino 4-butylamino 6-diéthylamino pyrimidine 3-oxyde**

ETAPE 1

***2-amino4-butylamino 6-chloro pyrimidine***

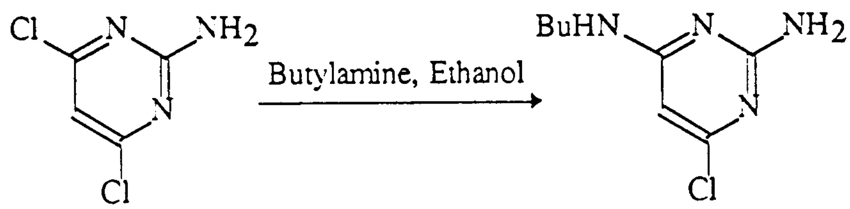


On place 10 g de 2-amino 4,6-dichloro pyrimidine en suspension dans 100 ml d'éthanol. On ajoute 11,15 g de butylamine. Le milieu réactionnel est porté au reflux pendant 2 heures puis évaporé à sec. On reprend dans 100 ml d'eau. Après 1 heure d'agitation, on filtre le précipité sur verre fritté puis on recristallise dans 80 ml d'eau/acétonitrile 6/4. On obtient 8,75 g de 2-amino 4-butylamino 6-chloro pyrimidine.

Rendement = 71 %

ANALYSES :

- Spectre de masse conforme
- Spectre RMN $^1$H conforme

Analyse élémentaire pour $C_8H_{13}N_4Cl$ ; M = 200,5

| | C | H | N | Cl |
|---|---|---|---|---|
| Calculé | 47,88 | 6,48 | 27,93 | 17,70 |
| Trouvé | 47,63 | 6,60 | 27,88 | 17,78 |

ETAPE 2

*2-amino 4-butylamino 6-chloro pyrimidine 3-oxyde*

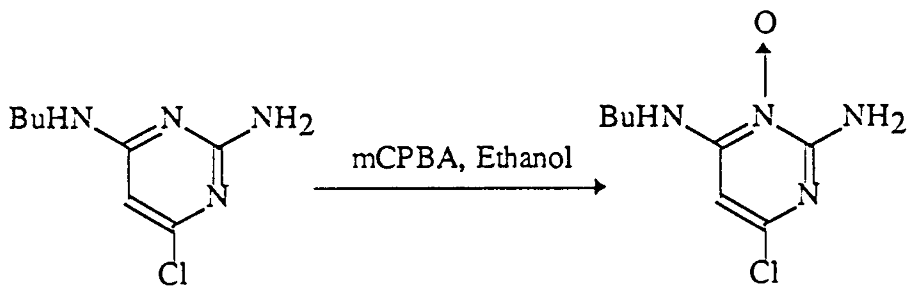


On solubilise 8,5 g de 2-amino 4-butylamino 6-chloro pyrimidine dans 150 ml d'éthanol. On additionne goutte à goutte 19,95 g d'acide métachloroperbenzoïque en solution dans 150 ml d'éthanol. Le milieu réactionnel est agité 2 heures à température ambiante. On ajoute 250 ml d'eau puis on évapore l'éthanol. Le pH est ajusté à 1 avec de l'acide chlorhydrique concentré. Après 1 heure d'agitation, le précipité est filtré sur verre fritté, rincé par 50 ml d'eau et éliminé. Le pH de la solution est ajusté à 8 par addition de soude. On extrait par 3 x 100 ml de dichlorométhane. La phase organique est séchée sur sulfate de sodium puis évaporée à sec. Le précipité obtenu est recristallisé dans 50 ml d'acétonitrile. On obtient 2,35 g de 2-amino 4-butylamino 6-chloropyrimidine 3-oxyde.

Rendement = 26%

ANALYSES :

- Spectre de masse conforme
- Spectre RMN $^1H$ conforme

Analyse élémentaire pour $C_8H_{13}N_4OCl$ ; M = 216,5

| | C | H | N | O | Cl |
|---|---|---|---|---|---|
| Calculé | 44,34 | 6,00 | 25,87 | 7,39 | 16,40 |
| Trouvé | 44,28 | 6,08 | 25,74 | 7,40 | 16,32 |

ETAPE 3

*2-amino 4-butylamino 6-diméthylamino pyrimidine 3-oxyde*

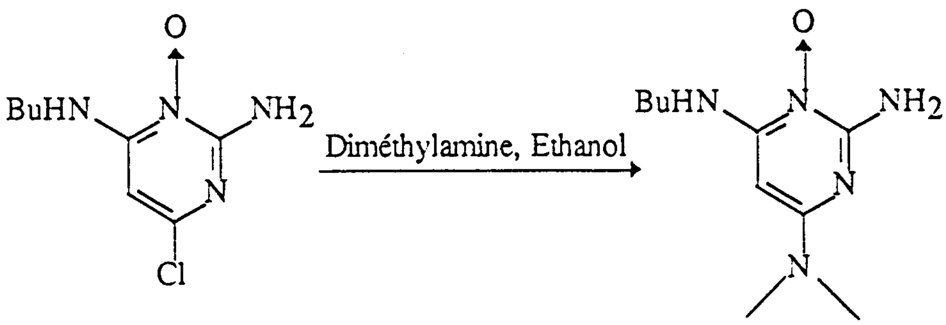


On place 1,5 g de 2-amino 4-butylamino 6-chloro pyrimidine 3-oxyde dans 10 ml d'éthanol. On ajoute 5 ml de diméthylamine en solution à 33% dans l'éthanol. Le milieu réactionnel est porté au reflux pendant 4 heures puis évaporé à sec. Le précipité obtenu est repris dans 20 ml d'eau. Le pH est ajusté à 8 par addition de soude. On extrait avec 3 x 50 ml de butanol. La phase butanolique est évaporée à sec. Le précipité est recristallisé dans 10 ml d'acétonitrile. On obtient 610 mg de 2-amino 4-butylamino 6-diméthylamino pyrimidine 3-oxyde.

Rendement: 39%

ANALYSES :

- Spectre de masse conforme
- Spectre RMN $^1H$ conforme

Analyse élémentaire pour $C_{10}H_{19}N_5O$ ; M = 225

| | C | H | N | O |
|---|---|---|---|---|
| Calculé | 53,33 | 8,44 | 31,11 | 7,11 |
| Trouvé | 53,50 | 8,50 | 31,11 | 7,30 |

EXEMPLE 10

**Synthèse du 2-amino 4-éthylamino 6-diméthylamino pyrimidine 3-oxyde**

ETAPE 1

***2−amino 4−éthylamino 6−chloro pyrimidine***

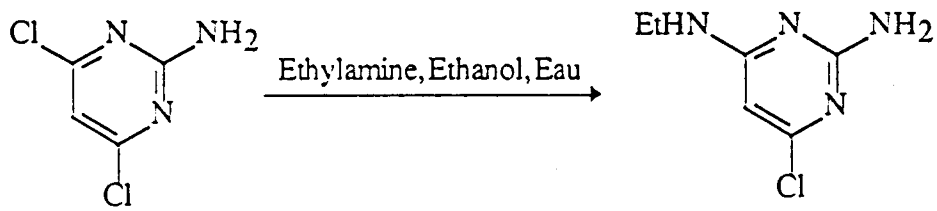


On place 10 g de 2-amino 4,6-dichloro pyrimidine en suspension dans 100 ml d'éthanol. On additionne 20,8 g d'éthylamine en solution à 33% dans l'eau. Le milieu réactionnel est porté au reflux pendant 2 heures puis évaporé à sec. Le précipité obtenu est repris dans 100 ml d'eau. Après 1 heure d'agitation, on filtre sur verre fritté. Le précipité est recristallisé dans 25 ml d'acétonitrile. On obtient 8,10 g de 2-amino 4-éthylamino 6-chloro pyrimidine.

Rendement = 77%

ANALYSES :

- Spectre de masse conforme
- Spectre RMN $^1$H conforme

Analyse élémentaire pour $C_6H_9N_4Cl$ ; M = 172,5

| | C | H | N | Cl |
|---|---|---|---|---|
| Calculé | 41,74 | 5,22 | 32,46 | 20,58 |
| Trouvé | 41,81 | 5,28 | 32,56 | 20,50 |

ETAPE 2

***2-amino 4-éthylamino 6-chloro pyrimidine 3-oxyde***

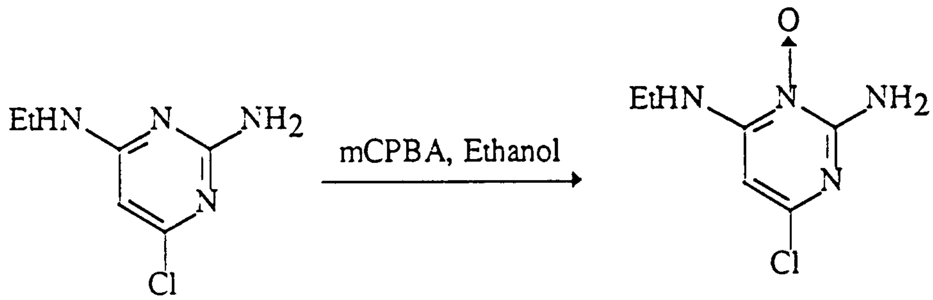


On place 8 g de 2-amino 4-éthylamino 6-chloro pyrimidine en suspension dans 150 ml d'éthanol. On additionne goutte à goutte 21,80 g d'acide métachloroperbenzoïque en solution dans 150 ml d'éthanol. Après 2 heures d'agitation, le milieu réactionnel est concentré au tiers au rotavapor. Le précipité est filtré sur verre fritté, lavé par 2 x 50 ml d'éther éthylique puis recristallisé dans 60 ml d'un mélange acétonitrile-éthanol 9/1. On obtient 3,05 g de 2-amino 4-éthylamino 6-chloro pyrimidine 3-oxyde.

Rendement = 35 %

ANALYSES :

- Spectre de masse conforme
- Spectre RMN $^1H$ conforme

ETAPE 3

***2-amino 4-éthylamino 6-diméthylamino pyrimidine 3-oxyde***

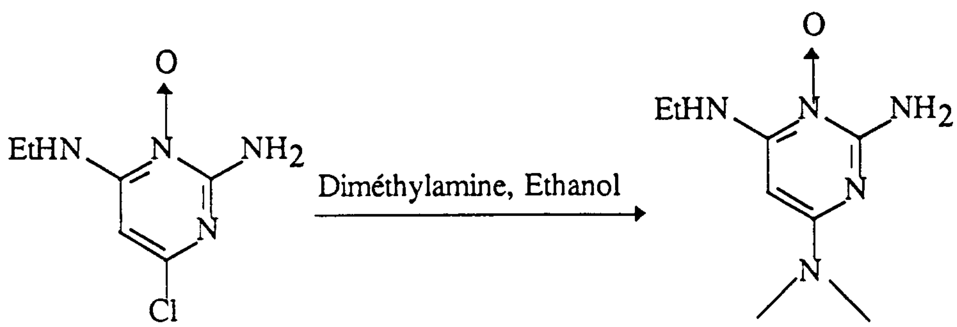


On place 3 g de 2-amino 4-éthylamino 6-chloro pyrimidine 3-oxyde dans 20 ml d'éthanol. On additionne 10 ml de diméthylamine en solution à 33% dans l'éthanol. Le milieu réactionnel est porté au reflux 4 heures puis évaporé à sec. On reprend dans 25 ml d'eau puis on ajuste le pH à 8 par addition de soude. On extrait par 4 x 50 ml de dichlorométhane. La phase organique est séchée sur sulfate de sodium, filtrée sur papier, évaporée à sec. Le précipité est recristallisé dans 26 ml d'un mélange acétonitrile-eau 95,75/4,25. On obtient 1,20 g de 2-amino 4-éthylamino 6-diméthylamino pyrimidine 3-oxyde.

Rendement = 38%

ANALYSES :

- Spectre de masse conforme
- Spectre RMN $^1$H conforme

Analyse pour $C_8H_{15}N_5O$ ; M = 197

| | C | H | N | O |
|---|---|---|---|---|
| Calculé | 48,73 | 7,61 | 35,53 | 8,12 |
| Trouvé | 48,79 | 7,64 | 35,52 | 8,29 |

EXEMPLE 11

**Synthèse du 2-amino 4-propylamino 5-chloro 6-diméthylamino pyrimidine 3-oxyde**

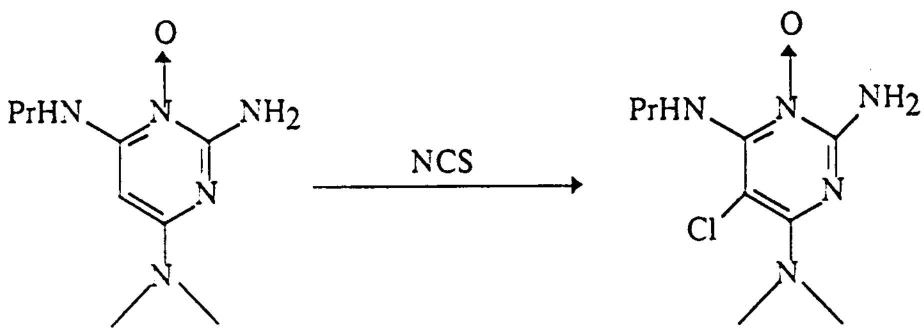


On place 1 g de 2-amino 4-propylamino 6-diméthylamino pyrimidine 3-oxyde de l'exemple 1 en solution dans 15 ml d'éthanol. On ajoute 0,75 g de N-chlorosuccinimide en solution dans 35 ml d'éthanol, goutte à goutte à température ambiante. Après 4 heures d'agitation, le milieu réactionnel est évaporé à sec. L'huile obtenue est reprise dans 10 ml d'eau. On amène le pH à 8 par addition de soude puis on extrait par 3 x 20 ml de butanol. La phase butanolique est évaporée à sec. Le résidu est recristallisé dans 5 ml d'acétonitrile. On obtient 0,7 g de 2-amino 4-propylamino 5-chloro 6-diméthylamino pyrimidine 3-oxyde.

Rendement = 60%

ANALYSES :

- Spectre de masse conforme
- Spectre RMN $^1$H conforme

Analyse élémentaire pour $C_9H_{16}N_5O$ Cl ; M = 245,5

| | C | H | N | O | Cl |
|---|---|---|---|---|---|
| Calculé | 43,99 | 6,52 | 28,51 | 6,52 | 14,46 |
| Trouvé | 44,19 | 6,54 | 28,46 | 6,76 | 14,36 |

EXEMPLES DE FORMULATION

**EXEMPLE 1**

On prépare la composition suivante :

| | | | |
|---|---|---|---|
| - 2-amino 4-propylamino 6-diméthylamino pyrimidine 3-oxyde | | 3,0 | g |
| - Ethanol à 95° | | 30,0 | g |
| - Eau | qsp | 100,0 | g |

Cette composition se présente sous la forme de lotion.

**EXEMPLE 2**

On prépare la composition suivante :

| | | | |
|---|---|---|---|
| - 4-amino 2-propylamino 6-diméthylamino pyrimidine 3-oxyde | | 2,0 | g |
| - Propylèneglycol | | 20,0 | g |
| - Ethanol | | 30,0 | g |
| - Eau | qsp | 100,0 | g |

Cette composition se présente sous la forme de lotion.

1 à 2 ml de ces lotions sont appliqués sur les zones alopéciques du cuir chevelu, une ou deux fois par jour, pendant 4 mois de traitement.

**Revendications**

**1.** Composition pour freiner la chute des cheveux et pour induire et stimuler leur croissance, caractérisée par le fait qu'elle contient dans un milieu physiologiquement acceptable, au moins un composé de formule:

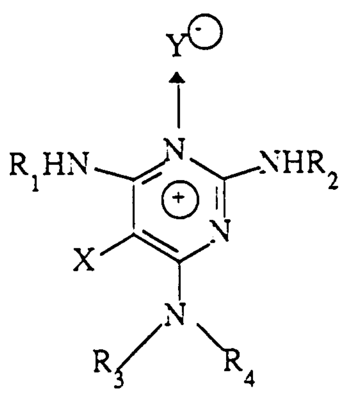

(I)

dans laquelle :

$R_1$ et $R_2$, indépendamment l'un de l'autre, désignent un atonie d'hydrogène ou un radical alkyle en $C_1$-

$C_8$, à condition qu'ils ne désignent pas simultanément un atome d'hydrogène;
$R_3$ et $R_4$, indépendamment l'un de l'autre, désignent un atome d'hydrogène, un radical alkyle en $C_1$-$C_8$ ou forment avec l'atome d'azote attaché à la position 6 du cycle pyrimidine, un hétérocycle en $C_3$-$C_8$, à condition qu'ils ne désignent pas simultanément un atome d'hydrogène;
X désigne un atome d'hydrogène ou un halogène;
Y désigne O ou $OSO_3$;
ainsi que ses sels d'addition d'acides physiologiquement acceptables.

2. Composition selon la revendication 1, caractérisée par le fait que dans la formule (I), les radicaux alkyle en $C_1$-$C_8$ sont choisis parmi méthyle, éthyle, n-propyle, isopropyle, n-butyle, tertiobutyle, hexyle, octyle, éthyl-2 hexyle; l'atome d'halogène est le chlore ou le brome; les groupements hétérocycles en $C_3$-$C_8$ sont choisis parmi morpholino, pipéridino, pyrrolidino, pipérazino, N-alkyl 4′-pipérazino dans lequel le groupement alkyle en position 4' contient de 1 à 6 atomes de carbone.

3. Composition selon la revendication 1, caractérisée par le fait que le composé de formule (I) est le 2-amino 4-propylamino 6-diméthylamino pyrimidine 3-oxyde ou l'un de ses sels d'addition d'acides physiologiquement acceptables.

4. Composition selon la revendication 1, 2 ou 3, caractérisée par le fait qu'elle se présente sous forme d'onguent, de teinture, de crème, de pommade, de poudre, de timbre, de tampon imbibé, de solution, d'émulsion, de dispersion vésiculaire, de lotion, de gel, de spray ou de suspension anhydre ou aqueuse en vue de son application pharmaceutique.

5. Composition selon la revendication 4, caractérisée par le fait que le composé de formule (I) est présent dans des concentrations comprises entre 0,1 et 10% en poids par rapport an poids total de la composition.

6. Composition cosmétique selon la revendication 1, 2 ou 3, caractérisée par le fait qu'elle se présente sous forme de lotion, de gel, de savon, de shampooing, d'aérosol ou de mousse et qu'elle contient, dans un support cosmétiquement acceptable, au moins un composé de formule (I) à une concentration comprise entre 0,01 et 5% en poids.

7. Composition selon l'une quelconque des revendications 1 à 6, caractérisée par le fait qu'elle contient en outre des agents hydratants et des agents antiséborrhéiques.

8. Composition selon l'une quelconque des revendications 1 à 7, caractérisée par le fait qu'elle contient également des agents améliorant encore l'activité des composés de formule (I) au niveau de la repousse et/ou du freinage de la chute des cheveux.

9. Composition selon la revendication 8, caractérisée par le fait qu'elle contient à titre d'agents améliorant encore l'activité de la repousse et/ou du freinage de la chute des cheveux, des esters d'acide nicotinique, des agents anti-inflammatoires stéroïdiens ou non-stéroïdiens, des rétinoïdes, des agents antibactériens, des agents antagonistes du calcium, des hormones, des agents anti-androgènes, des capteurs de radicaux OH, des oligosaccharides estérifiés, des dérivés d'acide hexosaccharíques, des inhibiteurs de glycosidase, des inhibiteurs de glycosaminoglycanases et protéoglycanases, des inhibiteurs de tyrosine kinase.

10. Composition selon la revendication 9, caractérisée par le fait qu'elle contient à titre de composés améliorant encore l'activité sur la repousse et/ou le freinage de la chute des cheveux, des composés choisis parmi le Diazoxide, la Spiroxazone, les phospholipides, les acides linoléique et linolénique, l'acide salicylique et ses dérivés, les acides hydroxycarboxyliques ou cétocarboxyliques, leurs esters, les lactones et leurs sels correspondants, l'anthraline, les caroténoïdes, les acides eicosatétraïnoïque-5,8,11,14, et eicosatriynoïque-5,8,11, leurs esters et amides.

11. Composition selon l'une quelconque des revendications 1 à 10, caractérisée par le fait qu'elle contient également des agents tensioactifs choisis parmi les agents tensio-actifs non-ioniques et amphotères.

**12.** Composition selon l'une quelconque des revendications 1 à 11, caractérisée par le fait que le milieu physiologiquement acceptable est constitué par de l'eau, un mélange d'eau et d'un ou plusieurs solvant(s) organique(s) ou par un mélange de solvants organiques, les solvants organiques étant pharmaceutiquement ou cosmétiquement acceptables.

**13.** Composition selon la revendication 12, caractérisée par le fait que les solvants sont choisis parmi les alcools inférieurs en $C_1$-$C_4$, les alkylèneglycols et les alkyléthers de mono- et de dialkylèneglycol.

**14.** Composition selon l'une quelconque des revendications 1 à 13, caractérisée par le fait que le milieu physiologiquement acceptable est épaissi au moyen d'agents épaississants et/ou gélifiants et contient des agents conservateurs, des agents stabilisants, des agents régulateurs de pH, des agents modificateurs de pression osmotique, des agents émulsifiants, des filtres UV-A et UV-B, des agents antioxydants.

**15.** Composés répondant à la formule suivante :

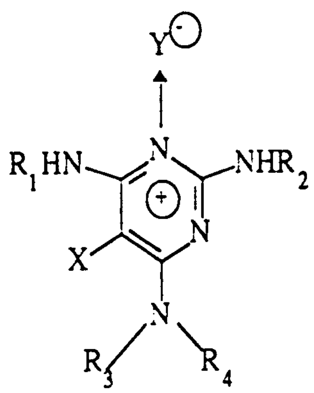

(I')

dans laquelle : X désigne un atome d'hydrogène ou d'halogène;
Y désigne O ou $OSO_3$;
$R_1$ et $R_2$, indépendamment l'un de l'autre, désignent un hydrogène ou un alkyle en $C_1$-$C_8$ à condition qu'ils ne désignent pas simultanément l'hydrogène;
$R_3$ et $R_4$, indépendamment l'un de l'autre, désignent un hydrogène, un alkyle en $C_1$-$C_8$ ou forment avec l'azote attaché à la position 6 du cycle pyrimidine un hétérocycle en $C_3$-$C_8$, sous réserve que $R_1$ et $R_2$ désignent simultanément un radical alkyle en $C_1$-$C_8$; ainsi que leurs sels d'addition d'acides physiologiquement acceptables.

**16.** Composés de formule (I') tels que définis dans la revendication 15, pour utilisation comme substances thérapeutiques.

**17.** Utilisation des composés de formule (I) tels que définis dans l'une quelconque des revendications 1 à 3, pour la préparation d'un médicament destiné au traitement de l'alopécie, la pelade, la chute des cheveux ou les dermatites desquamantes.

**18.** Procédé de traitement cosmétique des cheveux et du cuir chevelu, caractérisé par le fait que l'on applique une composition telle que définie dans l'une quelconque des revendications 1 à 14.

## Claims

**1.** A composition for slowing down the loss of hair and for inducing and stimulating its growth, which contains, in a physiologically acceptable medium, at least one compound of formula:

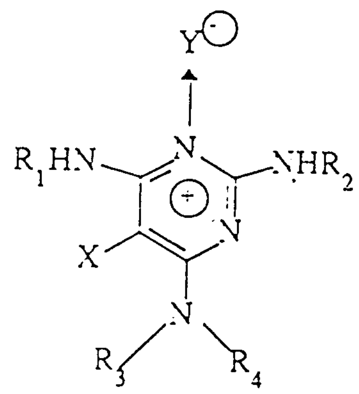

(I)

in which:

$R_1$ and $R_2$, independently of each other, denote a hydrogen atom or a $C_1$-$C_8$ alkyl radical, on condition that they do not simultaneously denote a hydrogen atom;

$R_3$ and $R_4$, independently of each other, denote a hydrogen atom or a $C_1$-$C_8$ alkyl radical or form, with the nitrogen atom attached to position 6 of the pyrimidine ring, a $C_3$-$C_8$ heterocyclic ring, on condition that they do not simultaneously denote a hydrogen atom;

X denotes a hydrogen atom or a halogen;

Y denotes O or $OSO_3$; and its addition salts of physiologically acceptable acids.

**2.** The composition as claimed in claim 1, wherein in formula (I) the $C_1$-$C_8$ alkyl radicals are chosen from methyl, ethyl, n-propyl, isopropyl, n-butyl, tert-butyl, hexyl, octyl and 2-ethylhexyl; the halogen atom is chlorine or bromine, the $C_3$-$C_8$ heterocyclic groups are chosen from morpholino, piperidino, pyrrolidino, piperazino and N-4'-alkylpiperazino in which the alkyl group in position 4' contains from 1 to 6 carbon atoms.

**3.** The composition as claimed in claim 1, wherein the compound of formula (I) is 2-amino-4-propylamino-6-dimethylaminopyrimidine 3-oxide or one of its addition salts of physiologically acceptable acids.

**4.** The composition as claimed in claim 1, 2 or 3, wherein it is in the form of ointment, tincture, cream, pomade, powder, plaster, saturated pad, solution, emulsion, vesicular dispersion, lotion, gel, spray or anhydrous or aqueous suspension with a view to its pharmaceutical application.

**5.** The composition as claimed in claim 4, wherein the compound of formula (I) is present in concentrations of between 0.1 and 10% by weight relative to the total weight of the composition.

**6.** The cosmetic composition as claimed in claims 1, 2 or 3, wherein it is in the form of lotion, gel, soap, shampoo, aerosol or foam and wherein it contains, in a cosmetically acceptable carrier, at least one compound of formula (I) in a concentration of between 0.01 and 5 % by weight.

**7.** The composition as claimed in any one of claims 1 to 6, wherein it additionally contains hydrating agents and antiseborrheic agents.

**8.** The composition as claimed in any one of claims 1 to 7, wherein it also contains agents which further improve the activity of the compounds of formula (I) in respect of the regrowth and/or slowing down of the loss of hair.

**9.** The composition as claimed in claim 8, wherein it contains, as agents which further improve the activity of the regrowth and/or of the slowing down of hair loss, nicotinic acid esters, steroidal or nonsteroidal antiinflammatory agents, retinoids, antibacterial agents, calcium antagonistic agents, hormones, antiandrogenic agents, OH radical scavengers, esterified oligosaccharides, hexasaccharic acid derivatives, glycosidase inhibitors, glycosaminoglycanase and proteoglycanase inhibitors and tyrosine kinase inhibitors.

**10.** The composition as claimed in claim 9, wherein it contains as compounds further improving the activity on the regrowth and/or on the slowing down of the loss of hair, compounds chosen from diazoxide, spiroxazone, phospholipids, linoleic and linolenic acids, salicylic acid and its derivatives, hydroxycarboxylic or ketocarboxylic acids, their esters, lactones and their corresponding salts, anthralin, carotenoids and 5,8,11,14-eicosatetraynoic and 5,8,11-eicosatriynoic acids, their esters and amides.

**11.** The composition as claimed in any one of claims 1 to 10, wherein it also contains surface-active agents chosen from nonionic and amphoteric surface-active agents.

**12.** The composition as claimed in any one of claims 1 to 11, wherein the physiologically acceptable medium consists of' water, a mixture of water and one or more organic solvent(s) or of a mixture of organic solvents, the organic solvents being pharmaceutically or cosmetically acceptable.

**13.** The composition as claimed in claim 12, wherein the solvents are chosen from $C_1$-$C_4$ lower alcohols, alkylene glycols and alkyl ethers of mono- and dialkylene glycol.

**14.** The composition as claimed in any one of claims 1 to 13, wherein the physiologically acceptable medium is thickened by means of thickening and/or gelling agents and contains preserving agents, stabilizers, pH regulators, osmotic pressure modifiers, emulsifiers, UV-A and UV-B filters and antioxidants.

**15.** Compounds corresponding to the following formula:

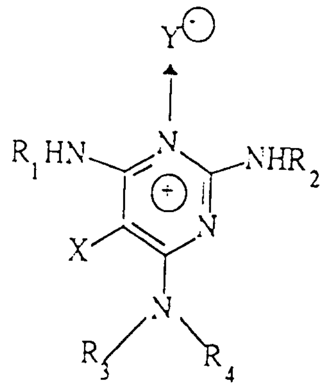

(I')

in which:
X denotes a hydrogen or halogen atom;
Y denotes O or $OSO_3$,
$R_1$ and $R_2$, independently of one another, denote a hydrogen or a $C_1$-$C_8$ alkyl, on condition that they do not simultaneously denote hydrogen;
$R_3$ and $R_4$, independently of each other, denote a hydrogen or a $C_1$-$C_8$ alkyl or form, with the nitrogen attached to position 6 of the pyrimidine ring, a $C_3$-$C_8$ heterocyclic ring provided that $R_1$ and $R_2$ simultaneously denote a $C_1$-$C_8$ alkyl radical; and their addition salts of physiologically acceptable acids.

**16.** Compounds of formula (I') as defined in claim 5, for use as therapeutic substances.

**17.** Use of the compounds of formula (I) as defined in any one of claims 1 to 3, for the preparation of a medication intended for the treatment of alopecia, pelade, hair loss or desquamative dermatitis.

**18.** Process for cosmetic treatment of hair and of the scalp, wherein a composition as defined in any one of claims 1 to 14 is applied.

## Patentansprüche

**1.** Zusammensetzung zum Vermindern des Haarausfalls und zum Auslösen und Anregen von deren Wachstum,

dadurch **gekennzeichnet**, daß
sie in einem physiologisch zulässigen Milieu mindestens eine Verbindung der Formel:

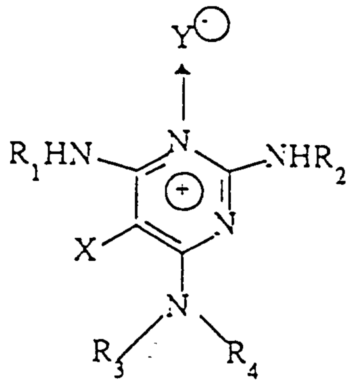

(I)

worin gilt:
$R_1$ und $R_2$ bedeuten, unabhängig voneinander, ein Wasserstoffatom oder einen $C_{1-8}$-Alkylrest, mit der Maßgabe, daß sie nicht gleichzeitig ein Wasserstoffatom darstellen;
$R_3$ und $R_4$ bedeuten, unabhängig voneinander, ein Wasserstoffatom, einen $C_{1-8}$-Alkylrest oder bilden zusammen mit dem an die Position 6 des Pyrimidinrings gebundenen Stickstoffatom einen $C_{3-8}$-Heterozyklus, mit der Maßgabe, daß sie nicht gleichzeitig ein Wasserstoffatom darstellen;
X bedeutet ein Wasserstoff- oder Halogenatom;
Y bedeutet O oder $OSO_3$,
sowie ihre physiologisch zulässigen Säureadditionssalze enthält.

2. Zusammensetzung gemäß Anspruch 1,
dadurch **gekennzeichnet**, daß
in der Formel (I) die $C_{1-8}$-Alkylreste aus Methyl-, Ethyl-, n-Propyl-, Isopropyl-, n-Butyl-, t-Butyl-, Hexyl-, Octyl-, 2-Ethylhexyl-Resten, das Halogenatom aus Chlor oder Brom und die heterozyklischen $C_{3-8}$-Gruppierungen aus Morpholino-, Piperidino-, Pyrrolidino-, Piperazino- und aus 4'-N-Alkylpiperazino-Gruppenausgewählt sind, in welcher die Alkylgruppe in Position 4' 1 bis 6 Kohlenstoffatome enthält.

3. Zusammensetzung gemäß Anspruch 1,
dadurch **gekennzeichnet**, daß
die Verbindung der Formel (I) 2-Amino-4-propylamino-6-dimethylaminopyrimidin-3-oxid oder eines seiner physiologisch zulässigen Säureadditionssalze ist.

4. Zusammensetzung gemäß Anspruch 1, 2 oder 3,
dadurch **gekennzeichnet**, daß
sie in Form einer Salbe, Tinktur, Creme, Pomade, eines Puders, Tupfers, getränkten Tampons, einer Lösung, Emulsion, bläschenartigen Dispersion, Lotion, eines Gels, eines Spray-Produkts oder einer wasserfreien oder wässrigen Suspension im Hinblick auf ihre pharmazeutische Anwendung vorliegt.

5. Zusammensetzung gemäß Anspruch 4,
dadurch **gekennzeichnet**, daß
die Verbindung der Formel (I) in Konzentrationen von 0,1 bis 10 Gew.%, bezogen auf das Gesamtgewicht der Zusammensetzung, vorhanden ist.

6. Kosmetische Zusammensetzung gemäß Anspruch 1, 2 oder 3,
dadurch **gekennzeichnet**, daß
sie in Form einer Lotion, eines Gels, einer Seife, eines Shampoo, Aerosols oder Schaums vorliegt und, in einem kosmetisch geeigneten Trägermedium, mindestens eine Verbindung der Formel (I) in einer Konzentration von 0,01 bis 5 Gew.% enthält.

7. Zusammensetzung gemäß jedem der Ansprüche 1 bis 6,
dadurch **gekennzeichnet**, daß

sie zusätzlich hydratisierende Mittel und antiseborrheische Mittel enthält.

**8.** Zusammensetzung gemäß jedem der Ansprüche 1 bis 7,
dadurch **gekennzeichnet**, daß
sie auch Mittel enthält, die die Wirksamkeit der Verbindungen der Formel (I) auf der Ebene des Wachstumsanstoßes und/oder der Verminderung des Ausfallens von Haaren noch verbessern.

**9.** Zusammensetzung gemäß Anspruch 8,
dadurch **gekennzeichnet**, daß
sie als Mittel, die die Wirksamkeit des Wachstumsanstoßes und/oder der Verminderung des Ausfallens von Haaren noch verbessern, Nicotinsäureester, entzündungshemmende steroidale oder nicht-steroidale Mittel, Retinoide, antibakterielle Mittel, Calcium-Antagonisten, Hormone, antiandrogene Mittel, Fängermittel von OH-Radikalen, veresterte Oligosaccharide, Hexosaccharinsäurederivate, Inhibitoren von Glycosidase, Inhibitoren von Glucosaminoglycanasen und Proteoglycanasen, Inhibitoren von Tyrosin-Kinase enthält.

**10.** Zusammensetzung gemäß Anspruch 9,
dadurch **gekennzeichnet**, daß
sie als Verbindungen, die die Wirksamkeit auf den Wachstumsanstoß und/oder die Verminderung des Ausfallens von Haaren noch verbessern, Verbindungen enthält, die aus Diazoxid, Spiroxazon, Phospholipiden, Linol- und Linolensäuren, Salicylsäure und ihren Derivaten, Hydroxycarboxyl- oder Ketocarboxylsäuren, ihren Estern, ihren Lactonen und ihren entsprechenden Salzen, Anthralin, Carotenoiden, Eicosa-5,8,11,14-tetrain- und Eicosa-5,8,11-triinsäuren, ihren Estern und Amiden ausgewählt sind.

**11.** Zusammensetzung gemäß jedem der Ansprüche 1 bis 10,
dadurch **gekennzeichnet**, daß
sie auch oberflächenaktive Mittel enthält, die aus nichtionischen und amphoteren oberflächenaktiven Mitteln ausgewählt sind.

**12.** Zusammensetzung gemäß jedem der Ansprüche 1 bis 11,
dadurch **gekennzeichnet**, daß
das physiologisch zulässige Milieu aus Wasser, einer Mischung aus Wasser und einem oder mehreren organischen Lösungsmitteln oder aus einer Mischung organischer Lösungsmittel zusammengesetzt ist, wobei die organischen Lösungsmittel pharmazeutisch oder kosmetisch zulässige sind.

**13.** Zusammensetzung gemäß Anspruch 12,
dadurch **gekennzeichnet**, daß
die Lösungsmittel aus $C_{1-4}$-Niedrigalkoholen, Alkylenglycolen und Alkylethern von Mono- und Dialkylenglycol ausgewählt sind.

**14.** Zusammensetzung gemäß jedem der Ansprüche 1 bis 13,
dadurch **gekennzeichnet**, daß
das physiologisch geeignete Milieu mit einem verdickenden und/oder gelierenden Mittel verdickt ist und Konservierungsmittel, Stabilisierungsmittel,
Regulierungsmittel für den pH-Wert, Modifizierungsmittel für den osmotischen Druck, Emulgatoren, Filterstoffe für UV-A und UV-B, Antioxydantien enthält.

**15.** Verbindungen gemäß der folgenden Formel:

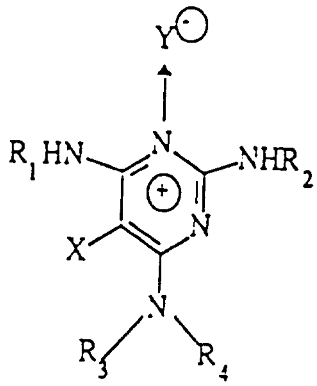

(I')

worin gilt:
X bedeutet ein Wasserstoff- oder Halogenatom;
Y bedeutet O oder $OSO_3$,
$R_1$ und $R_2$ beduten, unabhängig voneinander, ein Wassertoffatom oder eine $C_{1-8}$-Alkylgruppe, mit der Maßgabe, daß sie nicht gleichzeitig ein Wasserstoffatom darstellen;
$R_3$ und $R_4$ bedeuten, unabhängig voneinander, ein Wasserstoffatom, eine $C_{1-8}$-Alkylgruppe oder bilden mit dem an die Position 6 des Pyrimidinrings gebundenen Stickstoff einen $C_{3-8}$-Heterozyklus, mit der Maßgabe, daß $R_1$ und $R_2$ gleichzeitig einen $C_{1-8}$-Alkylrest darstellen;
sowie deren physiologisch zulässige Säureadditionssalze.

**16.** In Anspruch 15 definierte Verbindungen der Formel (I') zur Verwendung als therapeutische Substanzen.

**17.** Verwendung der in jedem der Ansprüche 1 bis 3 definierten Verbindungen der Formel (I) zur Herstellung eines Medikaments zur Behandlung der Alopezie, Pelade, des Ausfallens von Haaren oder von schuppenden Dermatitis-Erkrankungen.

**18.** Verfahren zur kosmetischen Behandlung der Haare und von behaarter Haut,
dadurch **gekennzeichnet**, daß
man eine in einem jeden der Ansprüche 1 bis 14 definierte Zusammensetzung auf diese aufträgt.